# EUROPEAN PATENT SPECIFICATION

(11) **EP 1 328 271 B1**
(45) Date of publication and mention of the grant of the patent: **25.06.2008**
(21) Application number: 01988583.9
(22) Date of filing: 29.10.2001
(51) Int. Cl.: A61K 31/40, A61K 31/425, A61P 25/22, A61K 38/17

(54) **TREATMENT OF NEUROLOGICAL AND NEUROPSYCHOLOGICAL DISORDERS**
BEHANDLUNG VON NEUROLOGISCHEN UND NEUROPSYCHOLOGISCHEN STÖRUNGEN
TRAITEMENT DE TROUBLES NEUROLOGIQUES ET NEUROPSYCHOLOGIQUES

(30) Priority: 27.10.2000 US 244036 P
(43) Date of publication of application: 23.07.2003
(60) Divisional application: 07122835.7
(73) Proprietor: Probiodrug AG, 06120 Halle/Saale (DE)
(72) Inventor: VON HÖRSTEN, Stephan, 30900 Wedemark (DE); KASK, Ants, EE 10912 Tallinn (EE); DEMUTH, Hans-Ulrich, 06114 Halle/Saale (DE); HOFFMANN, Matthias, 06688 Wengelsdorf (DE); KRUBER, Susanne, 53177 Bonn (DE); NGUYEN, Huu Phuc, 30625 Hannover (DE)
(74) Representative: Hoffmann, Matthias
(86) International application number: PCT/EP2001/012479
(87) International publication number: WO 2002/034243

(56) References cited:
- WO-A-00/53171
- WO-A-99/46272
- WO-A2-02/34242
- DE-U- 29 909 210
- WETTSTEIN J G ET AL: "Central nervous system pharmacology of neuropeptide Y." PHARMACOLOGY & THERAPEUTICS. ENGLAND MAR 1995, vol. 65, no. 3, March 1995 (1995-03), pages 397-414, XP001097134 ISSN: 0163-7258
- MUNGLANI R ET AL: "THE THERAPEUTIC POTENTIAL OF NEUROPEPTIDE Y ANALGESIC, ANXIOLYTIC AND ANTIHYPERTENSIVE" DRUGS, ADIS INTERNATIONAL LTD, AT, vol. 52, no. 3, September 1996 (1996-09), pages 371-389, XP002943797 ISSN: 0012-6667
- BADIA-ELDER N E ET AL: "Effects of neuropeptide Y (NPY) on ethanol intake and anxiety in high and low alcohol drinking (HAD1/LAD1) rats." ALCOHOLISM CLINICAL AND EXPERIMENTAL RESEARCH, vol. 24, no. 5 Supplement, May 2000 (2000-05), page 82A XP008006544 Scientific Meeting of the Research Society on Alcoholism;Santa Barbara, California, USA; June 24-29, 2000 ISSN: 0145-6008
- NUTT D.J.: 'Overview of diagnosis and drug treatments of anxiety disorders' CNS SPECTRUMS vol. 10, no. 1, 2005, pages 49 - 56
- 'Segmenting the anxiety disorders market' DATAMONITOR MARKET ANALYSIS EXPERTS, MARKET BRIEF March 2000, pages 1 - 39
- AUGUSTYNS K. ET AL: 'The unique properties of dipeptidyl-peptidase IV (DPP IV / CD26) and the therapeutic potential of DPP IV inhibitors' CURRENT MEDICINAL CHEMISTRY vol. 6, 1999, pages 311 - 327, XP000870290

## Description

### BACKGROUND OF THE INVENTION

### Field of the invention

The present invention relates to the maintanence or potentiation of endogenous neurological and neuropsychological effects of brain neuropeptide Y (NPY) systems by administration of inhibitors of dipeptidyl peptidase IV (DP IV). The invention relates further to the treatment of anxiety via a potentiation of NPY Y1 receptor mediated effects within the central nervous system (CNS).

### Background Art

### DP IV and NPY

DP IV (CD26; EC 3.4.14.5) is an exopeptidase with a triple functional role. DP IV is involved in the release of Xaa-Pro dipeptides from the N-terminus of polypeptides, circulating hormones and chemokines (Mentlein et al., 1999; Pauly et al., 1999), in T cell dependent immune responses (Kahne et al., 1999; Korom et al., 1997) and in metastasis (Cheng et al., 1998; 2000). DP IV selectively cleaves peptides after penultimate N-terminal proline and alanine residues. Endogenous substrates for this enzyme include the incretins, such as glucose-dependent insulinotropic polypeptides, like GIP and GLP-1. In the presence of DP IV, these hormones are enzymically degraded to inactive forms.

### Discovery of NPY

Neuropeptide Y (NPY), a 36 amino acid peptide belonging to the pancreatic polypeptide family, was first isolated from porcine brain in 1982 (Tatemoto and Mutt, 1982). NPY is present in all sympathetic nerves innervating the cardiovascular system and is the most abundant peptide in the brain and the heart. Additionally, in rats, but not in humans, NPY is also found extraneuronally in platelets and endothelium (Zukovska-Grojec et al., 1993). Originally, NPY was known as a potent vasoconstrictor and a neuromodulator. Released by stress, exercise, and myocardial ischemia, NPY has been implicated in coronary heart disease, congestive heart failure, and hypertension (Zukovska-Grojec ct al, 1998). More recently, because of the potent ability of NPY to stimulate food intake, it is suspected to play a role in obesity and diabetes (Kalra et al., 1999). Latest findings indicate that NPY is also a mitogen for rat aortic vascular smooth muscle cells (Zukovska-Grojec et al., 1999).

NPY-related research has focussed on at least three main directions: (1) Co-transmission and sympathetic vasoconstriction, because of its co-expression with noradrenaline; (2) neurotransmission and function within the CNS, because of potent consummatory effects; and (3) evolution of NPY, since NPY is one of the most highly conserved bio-active peptides known (Colmers and Wahlestedt, 1993; Lundberg, 1996; Wahlestedt and Reis, 1993; Wettstein et al., 1996). NPY acts on at least six receptors (Y1-Y6), with varying peptide pharmacology and distinct distribution in the CNS (Gehlert, 1998) (Tab. 1).

### Distribution of NPY, NPY receptor subtypes and mRNA

The distribution of NPY itself, NPY receptor protein and their mRNA within the CNS of human and rat brains has recently been reviewed (Dumont Y, Jacques D, St-Pierre, J.-A., Tong, Y., Parker, R., Herzog H. and Qurion, R., 2000; in Handbook of Chemical Neuroanatomy, Vol. 16: Peptide Receptors, Part 1; Quirion, R., Björklund, A. and Hökfeld, T., editors). A brief survey is given in Tab. 1.

NPY-containing neurons are evident in the nasal mucosa of various species including man, often associated with glandular acini and blood vessels (Baraniuk et. Al., 1990; Grunditz et. al., 1994). Stimulation of the parasympathetic nerve supply to the nasal mucosa (vidian nerve) in dogs increases blood flow in the region and causes mainly atropine resistance. Intravenous administration of NPY reduces vasodilitation due to parasympathetic nerve stimulation, an effect that was not mimicked by the NPY Y1- selective agonist [Leu31, Pro34]NPY, but was mimicked by administration of the NPY Y2receptor agonist N-acetyl[Leu28,Leu31JNPY(24-36) (Lacroix et al., 1994). This is consistent with a prejunctional NPY Y2- like receptor-mediated inhibition of transmitter release from parasympathetic nerve terminals.

### NPY receptor function

NPY is unarguably the most abundant neuropeptide discovered to date, with a wide distribution in the CNS and the peripheral nervous system (PNS). NPY forms a family of peptides together with peptide TY (PYY) (approximately 70% homology) and pancreatic polypeptide (PP) (approximately 50% homology); both NPY and PYY are extremely bio-active, whereas PP is generally much less active (Gehlert, 1998; Wahlestedt and Reis, 1993) (Tab. 2).

Two receptor subtypes of NPY have been called neuropeptide Y Y1 (postjunctional) and neuropeptide Y Y2 (prejunctional) on the basis of the different responses to a truncated analog of the related peptide YY-(13- 36), when compared with neuropeptide Y in *in vitro* assay systems (Wahlestedt et al., 1986). Activation of neuronal prejunctional NPY receptors generally inhibits nerve activity, reducing the release of neurotransmitters in response to nerve impulses and in response to local factors acting to relcase neurotransmitters (Wahlestedt et al., 1986). The prejunctional or neuropeptide Y Y2 receptor classification was based on actions of peptide YY (13-36) but in many systems this molecule, as well as neuropeptide Y-(13-36), does exhibit pressor activity (Rioux et al., 1986; Lundberg, et al., 1988; Potter et al., 1989). This has been interpreted by some to indicate that in some vascular beds there are two types of neuropeptide Y receptors (both neuropeptide Y Yj and neuropeptide Y2) on postjunctional membranes (Schwartz et al., 1989). However the lack of selectivity of these molecules may be due to retention of partial agonistic activity on Yj receptors, which permits them to evoke a reduced functional response. Previously, a 13-36 analog of neuropeptide Y, (Leu 17, Glu", Ala 21, Ala 22 , Glu 23 , LeU28, LeU31) neuropeptide Y- (13-36) (ANA neuropeptide Y-(13-36)) which displayed prejunctional activity equivalent to the whole neuropeptide Y molecule in studies *in vivo* was described (Potter et al., 1989).

Apart from these historically well-defined neuropeptide Y receptors the existence of a number of other subtypes (Y3, Y4, Y5 and Y6) has been suggested on a pharmacological basis (Michel et al., 1998) and details of the cloning of receptors corresponding to Y1, Y2, Y4 and Y5 have been published (Herzog et al., 1992; Gerald et al., 1995; Bard et al., 1995; Gerald et al., 1996) (Tab. 1). The distribution and physiological significance of these various receptor subtypes has yet to be defined. Although some controversy has existed about the selectivity of truncated forms of neuropeptide Y for one or other receptor subtype (Potter et al., 1989), the emerging picture supports the initial classification into pre-and postjunctional receptor subtypes. Cell lines have been developed which express specifically one neuropeptide Y receptor subtype and the development of receptor- selective analogs of neuropeptide Y has focussed mainly on binding characteristics in these cell lines (Sheikh et al., 1989; Aakerlund et al., 1990; Fuhlendorff et al., 1990). More recently, a cDNA encoding the neuropeptide Y Y1 receptor has been cloned and cell lines expressing the cloned receptor have been analyzed for both specific binding of neuropeptide Y analogs (Herzog et al., 1992) and functional responses elicited by specific analogs. From such binding studies, combined with subsequent studies *in vivo,* two analogs have been classified as acting specifically on the postjunctional neuropeptide Y Y1 receptor. These neuropeptide Y Y receptor selective analogs, (Pro 34) neuropeptide Y and (Leu", Pro 34) neuropeptide Y, mimic the action of neuropeptide Y in raising blood pressure, and also share similar binding to cell lines expressing only neuropeptide Y Y receptors e.g. the human neuroblastoma cell line SK-N-MC and fibroblast lines expressing the cloned neuropeptide Y Y, receptor (Herzog et al., 1992). Neither exhibits the neuropeptide Y Y2 receptor action an inhibition of cardiac vagal action *in vivo*, a manifestation of inhibition of acetylcholine release (Potter et al., 1991; Potter and McCloskey, 1992).

| **TABLE 1: DISTRIBUTION AND FUNCTION OF NPY RECEPTOR SUBTYPES WITHIN THE CNS** | | | | |
|---|---|---|---|---|
| **Receptor-subtype** | **CNS Expression** | **Function** | **Selective Agonist** | **Selective Antagonist or selectivity** |
| Y1 | Cortex, etc. | Anxiolysis, LHRH Release | Intact N - Terminus: [Leu31,Pro34]NPY | BIBP3226; BIBO 3304 |
| Y2 | Hippocampus, Hypothalamus | Antiamnestic | C-terminale End: PYY3-36; PYY13-36 | T4[NPY(33-36)]4; BIIE0246 |
| Y3 | Ncl. Tractus Solitarius (NTS) | Bradycardia, Hypotension | NPY>>PYY, [Leu31,Pro34]NPY | PYY - Insensitivity |
| Y4 | Dorsal vagal Complex (DVC) | Emetic | PP>>NPY, PYY | PP - Preferring |
| Y5 (a) | Hypothalamus | Feeding | NPY, PYY, [Leu31,Pro34]NPY | [Leu31,Pro34]NPY - sensitive, BIBP3226 - non-reversible |
| Y5 (b) or Y6 | Hypothalamus | ?; species specific | ? | ? |
| Tab. 1: NPY Receptor subtypes within the CNS; ? = unknown or not investigated | | | | |

The development of the high affinity, non-peptide NPY antagonists, BIBP3226 and BIBO3304, has facilitated the functional characterization of NPY receptors, as this compound shows selectivity for Y1R, being devoid of activity on at least Y2R, Y3R and Y4R (Doods et al., 1996). Recently, a two Y2 receptor antagonist has been described. One is a TASP-molecule (Grouzmann et al., 1997), the other a non-peptide antagonist (Wieland et al., 1999) and other non-peptide receptor specific compounds became available (Daniels et al., 1995). Thus, specific receptor blockade within the brain would allow the functional characterization of behavioral and physiological effects mediated by central NPY receptors. In addition, mice lacking the Y1R were generated and are available (Pedrazzini et al., 1998). Neurons showing NPY-like immunoreactivity and NPY receptor expression are abundant in the CNS (Tab. 1), and perhaps are most notably found in hypothalamic and so-called limbic structures, but are also co-localized with brain stem monoaminergic neurons and cortical GABA-ergic neurons (Chronwall, 1985; Dumont et al., 1996).

| TABLE 2: RECEPTOR SUBTYPES AND PEPTIDE SELECTIVITY | |
|---|---|
| Receptor subtype | Peptide Potency |
| Y1-like | |
| Y1 | NPY = PYY = Pro³⁴-NPY > PP > NPY₁₃₋₃₆ |
| Y4 | PP >> NPY = PYY = LP-NPY > NPY₁₃₋₃₆ |
| Y6 | NPY = PYY = Pro³⁴-NPY > NPY₁₃₋₃₆ > PP |

| Y2-like | |
|---|---|
| Y2 | NPY = PYY = NPY₁₃₋₃₆ > Pro³⁴-NPY > PP |

| Y5-like | |
|---|---|
| Y5 | NPY = PYY = Pro³⁴-NPY > NPY₁₃₋₃₆ > PP |

| Not cloned | |
|---|---|
| PP receptor | PP >> PYY = NPY |
| Y3 | NPY = Pro³⁴-NPY = NPY₁₃₋₃₆ >> PYY |
| PYY-preferring | PYY > NPY >> NPY₁₃₋₃₆ >> Pro³⁴-NPY |
| Tab. 2: Receptor subtypes and peptide selectivity according to Gehlert, 1998. | |

### NPY, anxiety and depression

Anxiolytic-like effects of NPY have been demonstrated using the elevated plus maze test (Montgomery), the punished drinking test (Vogel), and the punished responding test (Geller-Seifter), with potency and efficacy matching those of benzodiazepines (Griebel, 1999; Heilig et al., 1989; Wettstein et al., 1995). NPY increases sucrose intake, reduces ethanol intake, and reduces anxiety in high and low alcohol drinking (HAD1/ LAD1) rat lines (Badia-Elder, N.E. et al., Effects of neuropeptide Y (NPY) on ethanol intake and anxiety in high and low alcohol drinking (HAD1/LAD1) rats, Alcoholism Clinical and Experimental Research, 24 (2000) 82A.NPY acts anxiolytic-like on the response to novelty (Heilig and Murison, 1987; von Hörsten et al., 1998b), and produces anxiolytic-like effects on the elevated plus maze and other anxiety related tests (Wahlstedt and Reis, 1993; Wahlestedt et al., 1993). Interestingly, Y1 receptor antisense-treated rats showed marked anxiety-related behaviors, without alterations of locomotor activity and food intake (Wahlestedt et al., 1993). Additionally, in the Flinder rat strain, a genetic model of depression, Y1 receptor mRNA expression was decreased in different cortical regions and the dentate gyrus of the hippocampus, while Y2 receptor mRNA expression did not differ from controls (Caberlotto et al., 1998). Olfactory bulbectomy in the rat has been developed as a model of depression (Leonard and Tuite, 1981). In this model, most of the changes resemble those found in depressed patients (Song et al., 1996). A 7-day i.c.v. administration of NPY in olfactory bulbectomized rats attenuated behavioral and neurotransmitters deficits in this model (Song et al., 1996). NPY Y1, Y2, and possibly Y5 receptors, seem to be involved in the regulation of anxiety levels in rodents, with Y1-mediated effects being best characterized (Heilig et al., 1993; Kask et al., 1998b). It can be concluded, therefore, that endogenous NPY counteracts stress and anxiety (Heilig et al., 1994). Furthermore, these data suggest that the Y1 receptor subtype could be implicated in anxiety- and depression-related behaviors. Additionally, Kask et al. (1996) reported that i.c.v. injection of the Y1 antagonist, BIBP3226, produced anxiogenic-like effects in the elevated plus-maze test, without any locomotor deficit. This effect can be reproduced by the administration of BIBP3226 in the dorsal periaqueductal gray matter but not in the locus coeruleus or the paraventricular nucleus of the hypothalamus (Kask et al., 1998c). Moreover, BIBP3226 and GR231118 administered into the dorsal periaqueductal gray matter decreased the time spent in active social interaction in rats (Kask et al., 1998d). The brain regions which are important for the anti-stress action of NPY include but may not be limited to the amygdala (Sajdyk et al., 1999, Thorsell et al., 1999), locus coeruleus (Kask et al., 1998c) and dorsal periaqueductal gray (Kask et al., 1998a,b). Amygdala NPY is not released under low stress conditions since blockade of NPY Y₁R with BIBP3226 or BIBO3304 did not increase anxiety as measured in the elevated plus-maze and social interaction tests (Kask et al., 1998b; Sajdyk, 1999). Constant NPY-ergic tone, however, seems to exist in the dorsal periaqueductal gray matter, where the NPY Y₁R antagonist had anxiogenic like effects in both experimental anxiety models (Kask et al., 1998a,b). Thus, in certain brain regions, there may be a tonic regulation of anxiety via NPY systems.

### Neurological and psychophysiological effects of CNS NPY systems: Pleiotropy

Thus, numerous studies have addressed the physiological functions of NPY and its congeners in the CNS (for reviews see: Kalra and Crowley, 1992; Dumont et al., 1992; Stanley, 1993; Wahlestedt and Reis, 1993; Grundemar et al, 1993; Gehlert, 1994, 1998; Colmers and Bleakman, 1994; Wettstein et al, 1995; Heilig and Widerlow, 1995; Munglani et al., 1996; Inui, 1999; Bischoff and Michel, 1999; Vezzani et al., 1999) and demonstrated a broad range of effects. No pharmacological approaches exist, at present, to gain advantage of these various physiological functions.

### Current problems in the treatment of anxiety related disorders using benzodiazepines or NPY

The current methods for treatment of anxiety are accompanied by several problems:

The benzodiazepines that are commonly used as anxiolytic agents are unnatural compounds with a low or no selectivity. Beside their anxiolytic activity, the benzodiazepines show sedative and anti-epileptic effects and are suspected to influence muscle relaxation. Unfortunately, they are associated with a number of unwanted side effects, namely tiredness, sleepiness, lack of concentration, reduction of attentiveness and reactivity. Chronic application of benzodiazepines causes neurological disorders, like ataxia, dizziness, reflex loss, muscle and language disorders. A long-term treatment with benzodiazepines is predicted to entail dependency and addiction.

The direct i.c.v. administration of neuropeptide Y for the long-term treatment of anxiety in patients is not feasible.

WO 0053171 describes the use of metformin in the preparation of pharmaceutical compositions useful for inhibiting the enzyme dipeptidyl peptidase IV, in particular for increasing the plasma concentration of Glucagon-Like Peptide-1.

The registered German utility model, DE 299 09 210 U1, relates to isoleucyl-thiazolidide and isoleucyl-pyrrolidide, and salts thereof, and to pharmaceutical compositions containing at least one of the above listed compounds or a salt thereof, optionally, in combination with one or more pharmaceutically acceptable carriers and/or solvents, useful for lowering blood glucose in the serum of a mammal and for the treatment of Diabetes mellitus, impaired glucose tolerance, hyperlipidaemia, metabolic acidosis, diabetic neuropathy and nephropathy.

### Presence of DP IV and DP IV-like activity in the brain of mammals

Hartel-Schenk et al., 1990, studied the occurrence of DP IV during the development in Wistar rat organs on day 10, 16 and 21 of gestation and on day 1, 4, 8, 13, 21, 26 and 60 after birth comparing immunohistochemistry and activity histochemistry. In all investigated tissues, immunoreactivity with the polyclonal antibody appeared earlier than DP IV activity and was already present on day 10 of gestation in the plasma membranes of embryonic and extraembryonic (decidual) cells. At these and other sites, e.g. brain capillary endothelium and tracheal or bronchial epithelium, immunoreactivity with the polyclonal antibody decreased or disappeared after birth and enzyme activity never developed. Immunoreactivity with the monoclonal antibodies appeared later than that with the polyclonal antibody, and mostly in those structures where DP IV activity was subsequently found. The monoclonal antibody against epitope D showed a high reactivity in the epididymal duct, renal collecting ducts and in all domains of the hepatocyte plasma membrane, where neither DP IV activity nor immunoreactivity with the other antibodies were observed. Their results also suggest that DP IV might be present as a molecule before it becomes catalytically active and that immunoreactivity occurs at more sites than DP IV activity. By means of immunohistochemical techniques Bernstein et al, 1987, have investigated the presence of dipeptidyl aminopeptidase IV immunoreactivity in brain material derived from human fetuses, newborns and aged persons. It was revealed that the enzyme protein is abundantly present in the immature human CNS. On the contrary the adult human brain contains much less dipeptidyl aminopeptidase IV immunoreactivity. It is speculated that the enzyme might play an important role in neuronal proliferation and/or differentiation especially with regard to its possible action on certain neuronotrophic peptides (IGF II, growth hormone). Aminopeptidase M (APM), aminopeptidase A (APA), dipeptidyl peptidase IV (DP IV) and gamma-glutamyl transferase (GGT) were demonstrated histochemically in cryostat sections of the rat brain to show the reaction pattern of ependyma, choroid plexus and leptomeninges. GGT was only demonstrable in the cell membranes of ependymal cells and in the leptomeninges; however, APA, APM and DP IV showed a variable degree of activity in the capillary endothelium of the choroid plexus as well as in the leptomeninges. (Mitro & Lojda, 1988). Kato et al., 1979 found X-prolyl dipeptidyl-aminopeptidase activity in rat brain and examined the developmental changes at various ages. The total enzyme activity per brain increased until 4 weeks of age, and then decreased during maturation. Specific activity in young rat brain was higher than that in adult rat brain. The properties of the brain enzyme were different from those of pituitary and other tissues. Nässel et al., 2000, investigated the occurrence of proline-specific DP IV-activity in cockroach tissues. Partly purified DP IV-activity was characterized from the brain and midgut of L. maderae by using Gly-Pro-4-nitroanilide as a substrate. The highest activity was obtained from the membrane fraction of intestine; about 10 times less activity (per milligram protein) was obtained from brain membranes. It was reported that while 55°o of the total post-proline dipeptidyi-aminopeptidase activity in guinea-pig brain is associated with the soluble fraction of the cells, the remaining activity is widely distributed throughout the particulate fractions. A significant portion of this particulate activity is, however, associated with a synaptosomal membrane fraction. (O'Connor & O'Cuinn, 1986). Histochemical investigation by means of light and electron microscopy revealed the presence of dipeptidyl peptidase IV activity in Meissner corpuscles of macaque glabrous skin. The enzyme activity was found in fibroblast-like cells forming an incomplete capsule around the Meissner corpuscle. Distinct electron-dense reaction product due to dipeptidyl peptidase IV activity was consistently localized in the plasma membrane of specialized Schwann cells enveloping the unmyelinated portion of sensory axons. Their axolemma was devoid of dipeptidyl peptidase IV reaction product. (Dubovy, 1988)

De Bault & Mitro, 1994, examined the localization of membrane proteases glutamyl aminopeptidase (EAP), microsomal alanyl aminopeptidase (mAAP), dipeptidyl peptidase IV (DP IV) and gamma-glutamyl transpeptidase (gamma-GTP) in vessels of the rat subfornical organ (SFO), ependyma which cover the surface of the SFO, and adjacent brain structures. Results of enzyme histochemical reactions showed strong activity for EAP, mAAP, and gamma-GTP, but, in contrast to the above findings, absence of DP IV in microvessels of SFO. The ependyma which cover the SFO was positive for gamma-GTP, but negative for other studied proteases. Our results showed that the spectrum of enzymes in the majority of the vessels of SFO is similar to that of the microvessels of the adjacent brain tissue which were positive for EAP, mAAP, and gamma-GTP, but negative for DP IV. Using antibodies against and cDNA for DP IV. Hong et al., 1989, assessed the tissue distribution of DP IV by molecular approaches in rat. Immunoblot analysis demonstrated that DP IV is present in the kidney, lung, and small intestine at high levels, in the liver and spleen at moderate levels, and in the heart at low levels. The highest levels of mRNA for DP IV were detected in the kidney and small intestine as compared to moderate levels found in the lung, liver, and spleen. The lowest levels of DP IV mRNA were found in the stomach, testis, heart, muscle and brain.

### SUMMARY OF THE INVENTION

It is an object of the present invention to provide a medicament beneficial for neurological and psychophysiological effects, i.e. to provide a medicament for the treatment of anxiety.

In addition, it is an object of the present invention to overcome or reduce the above stated problems of the prior art by providing a pharmacological approach that results in a maintained or prolonged activity and/or effect of NPY in the brain of mammals.

These objects are solved by the use of an inhibitor of dipeptidyl peptidase IV (DP IV or CD26) for the production of a medicament for treating anxiety.

This results in the magnification of endogenous neurological or neuropsychological effects mediated by NPY Y1 receptors, resulting in a reduction of anxiety.

### BRIEF DESCRIPTION OF THE DRAWINGS

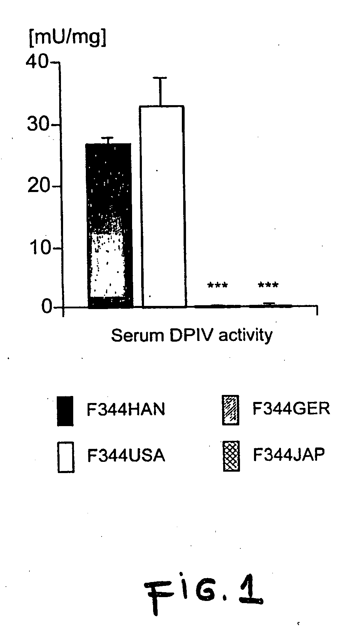

Figure 1 shows the DP IV enzymatic activity in serum of Fischer 344 (F344) rat substrains from Hannover (HAN), United States (USA), German (GER) and Japanese (JAP) breeders. The results are mean (±SEM) of 4-5 age-matched animals per genotype. Analysis of variance revealed a significant effect of "substrain" with F(3, 15): 50.4, p<0.0001. Asterisks indicate significant PLSD post hoc effects vs. "wild-type" F344USA and F344HAN substrains ("***" = p<0.0001).

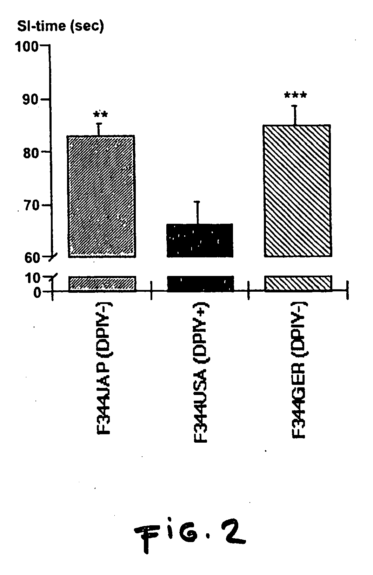

Figure 2 shows the time spent in active social interaction (SI) time in Fischer 344 (F344) rat substrains from Japanese (JAP), United States (USA) and German (GER) breeders. An increase of the SI time in the rat social interaction test of anxiety is interpreted as an anxiolytic-like response. The results are mean (±SEM) of 12 age-matched animals per genotype. Analysis of variance revealed a significant effect of "substrain" with F(2, 32): 8.8, p=0.0009. Asterisks indicate significant PLSD post hoc effects vs. "wild-type" F344USA rats ("**" = p<0.01; "***" = p<0.001).

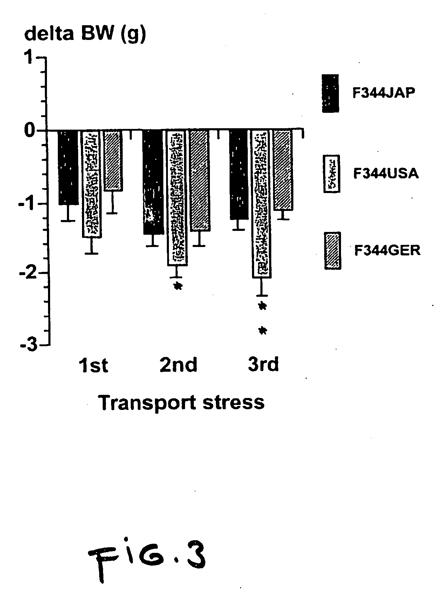

Figure 3 shows the change of body weight after stress on three consecutive days. On three consecutive days, age matched animals from Japanese (JAP), United States (USA) and German (GER) breeders were individually transported to a novel room and remained there for 1h. On the first day a novel cage containing sawdust was used and animals placed in a standard animal rack. On the second day procedure was the same except that the cage was without sawdust. The stress procedure on the third was the same as on day 2 except that the cage was placed on the bottom of the novel room. Analysis of variance for repeated measures revealed a significant effect of "substrain" with F(2, 30): 13.5, p=0.0004. Asterisks indicate significant PLSD post hoc effects one factor ANOVAs split by day vs. "wild-type" F344USA ("*" = p<0.05; "**" = p<0.01).

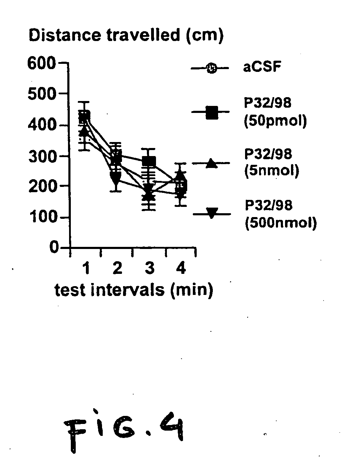

Figure 4 shows the effect of i.c.v. isoleucyl thiazolidine treatment on the distance traveled in four consecutive minutes of open field testing. Analysis of variance for repeated measures revealed no significant effect of treatment on this parameter of activity (F(3, 78): 0.7, p=0.5; n.s.).

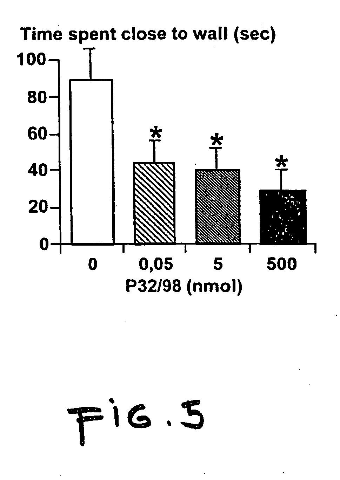

Figure 5 shows the effect of i.c.v. isoleucyl thiazolidine treatment on the time spent close to the wall as a sum of four consecutive minutes of open field testing. Analysis of variance revealed a significant effect of "treatment" with F(3, 26): 4.1, p=0.015. Asterisks indicate significant PLSD post hoc effects vs. "aCFS" controls ("*" = p<0.05).

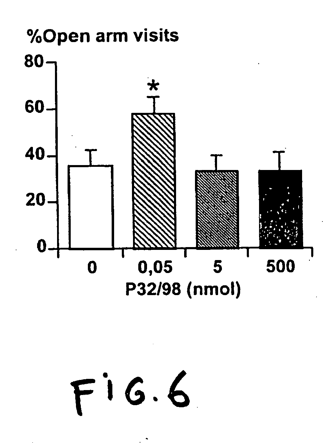

Figure 6 shows the effect of i.c.v. isoleucyl thiazolidine treatment on the percentage of time spent on the open arms of the elevated plus maze (EPM). Analysis of variance revealed a significant effect of "treatment" with F(3, 26): 3.0, p=0.048. Asterisks indicate significant PLSD post hoc effects vs. "aCFS" controls ("*" = p<0.05).

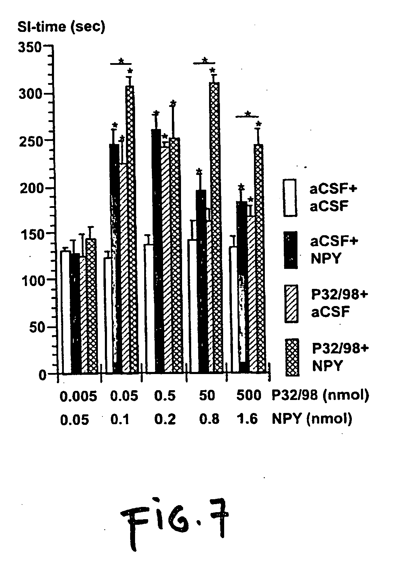

Figure 7 shows the effect of combined i.c.v. treatment using aCSF, isoleucyl thiazolidine and NPY in combinations at different dosages (isoleucyl thiazolidine: 5pmol-500nmol; NPY: 50pmol-1.6nmol). The time spent in active social interaction (SI-time) in the social interaction test of anxiety was measured. An increase of the SI-time is indicative for an anxiolytic like effect. After habituation to the testing procedure animals were repeatedly tested with randomly chosen treatment and always-new interaction partners. Tests were separated with at least 4 days from each other. For each test, spanning four groups of 5-6 animals per condition, analysis of variance revealed the following significant effects of "treatment" (aCSF+aCSF; aCSF+NPY; isoleucyl thiazolidine+aCSF; isoleucyl thiazolidine+NPY) from left to right: isoleucyl thiazolidine 5pmol+NPY 50pmol: F(3, 18): 0.25, p=0.8, n.s.; isoleucyl thiazolidine 50pmol+NPY 100pmol: F(3, 18): 22.4, p<0.0001; isoleucyl thiazolidine 500pmol+NPY 200pmol: F(3, 20): 8.6, p=0.007; isoleucyl thiazolidine 50nmol+NPY 0.8nmol: F(3, 20): 23.3, p<0.0001; and isoleucyl thiazolidine 500nmol+NPY 1.6nmol: F(3, 20): 11.2, p=0.0008. Asterisks indicate significant PLSD post hoc effects vs. "aCFS+aCSF" controls and as indicated by bars between aCSF+NPY vs. isoleucyl thiazolidine+NPY ("*" = p<0.05).

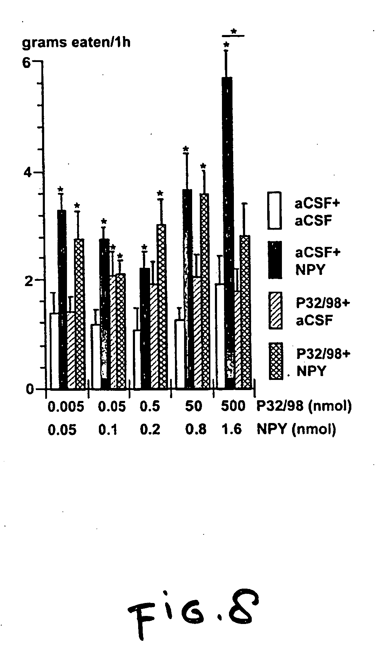

Figure 8 shows the effect of combined i.c.v. treatment using aCSF, isoleucyl thiazolidine and NPY in combinations at different dosages (isoleucyl thiazolidine: 5pmol-500nmol; NPY: 50pmol-1.6nmol). The amount of food eaten within 1h was measured. Animals were repeatedly tested with randomly chosen treatments. Tests were separated with at least 4 days from each other. For each test, spanning four groups of 5-6animals per condition, analysis of variance revealed the following significant effects of "treatment" (aCSF+aCSF; aCSF+NPY; isoleucyl thiazolidine+aCSF; isoleucyl thiazolidine+NPY) from left to right: isoleucyl thiazolidine 5pmol+NPY 50pmol: F(3, 18): 7.0, p=0.0025; ; isoleucyl thiazolidine 50pmol+NPY 100pmol: F(3, 20): 4.5, p=0:016; isoleucyl thiazolidine 500pmol+NPY 200pmol: F(3, 20): 4.4, p=0.015; isoleucyl thiazolidine 50nmol+NPY 0.8nmol: F(3, 20): 6.6, p=0.0027; and isoleucyl thiazolidine 500nmol+NPY 1.6nmol: F(3, 20): 13.7, p<0.0001. Asterisks indicate significant PLSD post hoc effects vs. "aCFS+aCSF" controls and as indicated by bars between aCSF+NPY vs. isoleucyl thiazolidine+NPY ("*" = p<0.05).

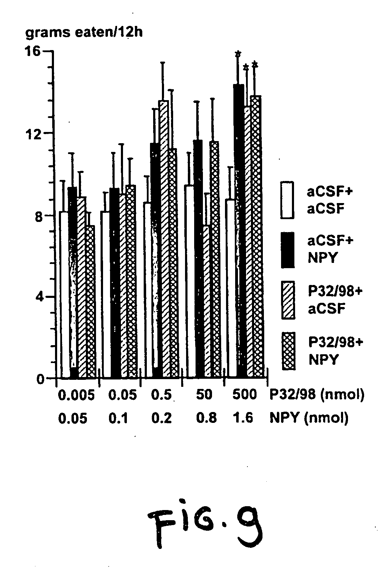

Figure 9 shows the effect of combined i.c.v. treatment using aCSF, isoleucyl thiazolidine and NPY in combinations at different dosages (isoleucyl thiazolidine: 5pmol-500nmol; NPY: 50pmol-1.6nmol). The amount of food eaten within 12h was measured. For each test, spanning four groups of 5-6 animals per condition, analysis of variance revealed the following significant effects of "treatment" (aCSF+aCSF; aCSF+NPY; isoleucyl thiazolidine+aCSF; isoleucyl thiazolidine+NPY) from left to right: isoleucyl thiazolidine 5pmol+NPY 50pmol: F(3, 18): 0.5, p=0.7, n.s.; isoleucyl thiazolidine 50pmol+NPY 100pmol: F(3, 20): 0.17, p=0.9, n.s.; isoleucyl thiazolidine 5OOpmol+NPY 200pmol: F(3, 20): 1.1, p=0.34, n.s.; isoleucyl thiazolidine 50nmol+NPY 0.8nmol: F(3, 20): 1.2, p=0.3; and isoleucyl thiazolidine 500nmol+NPY 1.6nmol: F(3, 20): 3.4, p=0.039. Asterisks indicate significant PLSD post hoc effects vs. "aCFS+aCSF" controls and as indicated by bars between aCSF+NPY vs. isoleucyl thiazolidine+NPY ("*" = p<0.05).

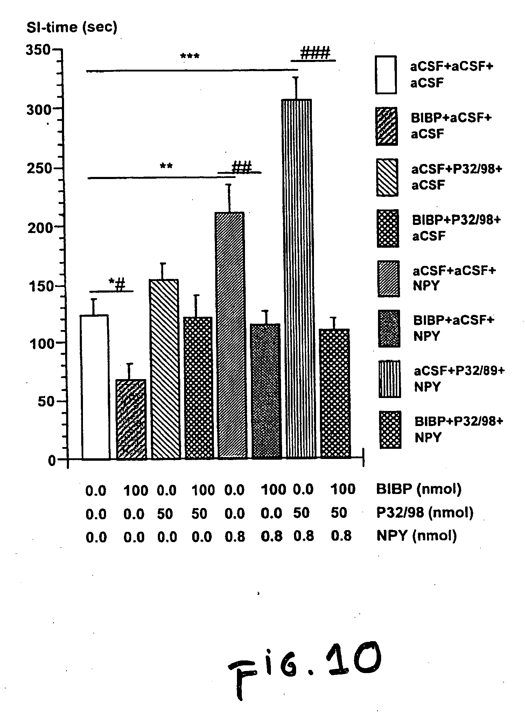

Figure 10 shows the effect of combined i.c.v. treatment using the Y1R antagonist BIBP3226, isoleucyl thiazolidine and NPY in combinations (BIBP3226: 100nmol; isoleucyl thiazolidine: 50nmol; NPY: 0.8nmol). The time spent in active social interaction (SI-time) in the social interaction test of anxiety was measured. An increase of the SI-time is indicative for an anxiolytic like effect. After habituation to the testing procedure animals were randomly assigned to i.c.v. treatment protocols and same treatment interaction partners. Tests spanned two consecutive with a total of 6-8 animals per treatment condition. Analysis of variance revealed a significant effect of "treatment" with F(7, 44): 33.6, p<0.0001 spanning the following groups: (1) aCSF+aCSF+aCSF; (2) BIBP +aCSF+aCSF; (3) aCSF+isoleucyl thiazolidine+aCSF; (4) BIBP +isoleucyl thiazolidine+aCSF; (5) aCSF+aCSF+NPY; (6) BIBP +aCSF+NPY; (7) aCSF+P32/89+NPY; (8) BIBP +isoleucyl thiazolidine+NPY. The level of significance in post hoc comparisons vs. controls (aCSF+aCSF+aCSF) is indicated by asterisks with "*" = p<0.05; "**" = p<0.01; "***" p<0.001) and vs. corressesponding antagonistic treatment (BIBP +n.n.) by "#" symbols with "#" = p<0.05; "##" = p<0.01; "###" = p<0.001). All data are presented as means ± S.E.M.

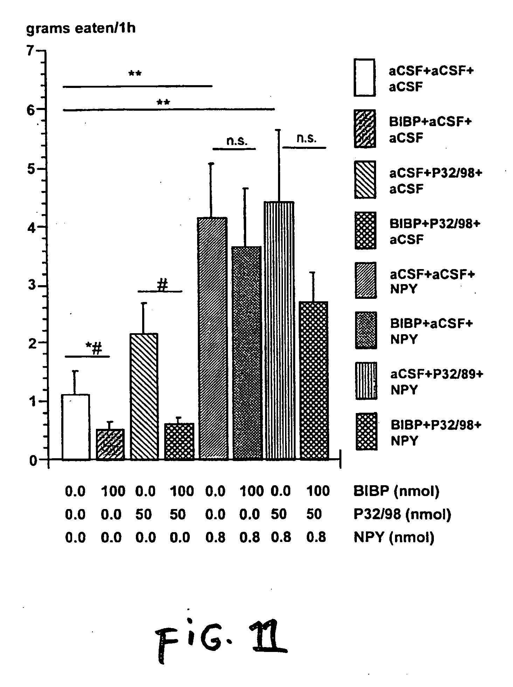

Figure 11 shows the effect of combined i.c.v. treatment on 1h food-intake using the Y1R antagonist BIBP3226, isoleucyl thiazolidine and NPY in combinations (BIBP3226: 100nmol; isoleucyl thiazolidine: 50nmol; NPY: 0.8nmol). After habituation to the testing procedure animals were randomly assigned to i.c.v. treatment protocols and same treatment interaction partners. Tests spanned two consecutive with a total of 6-8 animals per treatment condition. Analysis of variance revealed a significant effect of "treatment" with F(7, 44): 5.4, p<0.0002 spanning the following groups: (1) aCSF+aCSF+aCSF; (2) BIBP +aCSF+aCSF; (3) aCSF+isoleucyl thiazolidine+aCSF; (4) BIBP +isoleucyl thiazolidine+aCSF; (5) aCSF+aCSF+NPY; (6) BIBP +aCSF+NPY; (7) aCSF+P32/89+NPY; (8) BIBP +isoleucyl thiazolidine+NPY. The level of significance in post hoc comparisons vs. controls (aCSF+aCSF+aCSF) is indicated by asterisks with "*" = p<0.05; "**" = p<0.01; "***" = p<0.001) and vs. corresponding antagonistic treatment (BIBP +n.n.) by "#" symbols with "#" = p<0.05; "##" = p<0.01; "###" = p<0.001). All data are presented as means ± S.E.M.

### DETAILED DESCRIPTION OF THE INVENTION

In contrast to other proposed methods in the art, the present invention provides an orally available therapy with low molecular weight inhibitors of dipeptidyl peptidase IV. The instant invention represents a novel approach for the treatment of anxiety in mammals. It is user friendly, commercially useful and suitable for use in a therapeutic regime, especially concerning human disease.

Examples for orally available low molecular weight dipeptidyl peptidase IV inhibitors are agents such as, N-(N'-substituted glycyl)-2-cyanopyrrolidines, L-*threo*-isoleucyl thiazolidine, L-*allo*-isoleucyl thiazolidine, L-*threo*-isoleucyl pyrrolidine, L-*allo*-isoleucyl thiazolidine, and L-*allo*-isoleucyl pyrrolidine. They are described in US 6, 001, 155, WO 99/61431, WO 99/67278, WO 99/67279, DE 198 34 591, WO 97/40832, DE 196 16 486 C2, WO 98/19998, WO 00/07617, WO 99/38501, and WO 99/46272, the teachings of which are herein incorporated by reference in their entirety. The goal of these agents is to inhibit DP IV; and by doing so, to lower blood glucose levels thereby effectively treating hyperglycemia and the attendant disease associated with elevated levels of glucose in the blood.

DP IV is an enzyme that is an exopeptidase, which selectively cleaves peptides after penultimate N-terminal proline and alanine residues. Endogenous substrates for this enzyme include the incretins, such as glucose-dependent insulinotropic polypeptides, like GIP and truncated GLP-1. In the presence of DP IV, these hormones are enzymically reduced to inactive forms. The inactive form of GIP and GLP-1 cannot induce insulin secretion, thus blood glucose levels are elevated, especially in the hyperglycemic state. Elevated blood glucose levels have been associated with many different pathologies, including diabetes mellitus (Type 1 and 2) and the sequel accompanying diabetes mellitus.

It has also been discovered that DP IV plays a role in T-cell-mediated immune responses, for example, in organ transplantation. Inhibition of DP IV has been demonstrated to prolong cardiac allografts. Additionally, the inhibition of DP IV has contributed to the suppression of rheumatoid arthritis. DP IV has also been attributed a role in HIV's penetration into T-cells (T-helper cells).

These various effects of dipeptidyl peptidase IV inhibitors imply their impact on normal healthy tissues and organs, when they are used for the treatment of a pathologically altered tissue. The goal of the present invention is the development of brain targeted agents which maintain, increase or prolong the effect or activity of NPY and which display a high bioavailability and an exactly predictable activity time in the target tissue.

Examples for target specific, orally available low molecular weight agents are prodrugs of stable and unstable dipeptidyl peptidase IV inhibitors which comprise general formula A-B-C, whereby A represents an amino acid, B represents the chemical bond between A and C or an amino acid, and C represents an unstable or a stable inhibitor of dipeptidyl peptidase IV, respectively. They are described in WO 99/67278, WO 99/67279 the teachings of which are herein incorporated by reference in their entirety.

It is disclosed here that the administration of an inhibitor of the enzyme dipeptidyl peptidase (DP IV or CD 26) in the brain of mammals leads as a causal consequence to a reduced degradation of the neuropeptide Y (NPY). Such treatment will result in a reduction or delay in the decrease of the concentration of functional active NPY (1-36).

According to the present invention it has been found that the effect and/or activity of NPY in the brain of mammals, especially humans, can be maintained or prolonged by the administration of inhibitors of dipeptidyl peptidase IV (DP IV). Thereby, the degradation of NPY in the brain can be reduced. The instant invention especially represents a novel approach for the treatment of anxiety. It is user friendly, commercially useful and suitable for use in a therapeutic regime, especially concerning human disease. The finding that the administration of inhibitors of DP IV can prevent or slow down the degradation of NPY in the brain is particularly surprising for the following reasons: . Hong et al., 1989, assessed the tissue distribution of DP IV in rat tissues, using antibodies against and cDNA for DP IV. Immunoblot analysis demonstrated that DP IV is present in the kidney, lung, and small intestine at high levels, in the liver and spleen at moderate levels, and in the heart at low levels. The highest levels of mRNA for DP IV were detected in the kidney and small intestine as compared to moderate levels found in lung, liver, and spleen. The lowest levels of DP IV mRNA were found in the stomach, testis, heart, muscle and brain.

Hartel-Schenk et al., 1990, studied the occurrence of DP IV during the development in Wistar rat organs on day 10, 16 and 21 of gestation and on day 1.4, 8, 13, 21, 26 and 60 after birth comparing immunohistochemistry and activity histochemistry. In all investigated tissues, immunoreactivity with the polyclonal antibody appeared earlier than DP IV activity and was already present on day 10 of gestation in the plasma membranes of embryonic and extraembryonic (decidual) cells. At these and other sites, e.g. brain capillary endothelium and tracheal or bronchial epithelium, immunoreactivity with the polyclonal antibody decreased or disappeared after birth and enzyme activity never developed. Immunoreactivity with the monoclonal antibodies appeared later than that with the polyclonal antibody, and mostly in those structures where DP IV activity was subsequently found. The monoclonal antibody against epitope D showed a high reactivity in the epididymal duct, renal collecting ducts and in all domains of the hepatocyte plasma membrane, where neither DP IV activity nor immunoreactivity with the other antibodies were observed. Their results also suggest that DP IV might be present as a molecule before it becomes catalytically active and that immunoreactivity occurs at more sites than DP IV activity. By means of immunohistochemical techniques Bernstein et al, 1987, have investigated the presence of dipeptidyl aminopeptidase IV immunoreactivity in brain material derived from human fetuses, newborns and aged persons. It was revealed that the enzyme protein is abundantly present in the immature human CNS. On the contrary the adult human brain contains much less dipeptidyl aminopeptidase immunoreactivity. It is speculated that the enzyme might play an important role in neuronal proliferation and/or differentiation especially with regard to its possible action on certain neuronotrophic peptides (IGF II, growth hormone). De Bault & Mitro, 1994, examined the localization of membrane proteases glutamyl aminopeptidase (EAP), microsomal alanyl aminopeptidase (mAAP), dipeptidyl peptidase IV (DP IV) and gamma-glutamyl transpeptidase (gamma-GTP) in vessels of the rat subfornical organ (SFO), ependyma which cover the surface of the SFO, and adjacent brain structures. Results of enzyme histochemical reactions showed strong activity for EAP, mAAP, and gamma-GTP, but, in contrast to the above findings, absence of DP IV in microvessels of SFO. The ependyma which cover the SFO was positive for gamma-GTP, but negative for other studied proteases. Their results showed that the spectrum of enzymes in the majority of the vessels of SFO is similar to that of the microvessels of the adjacent brain tissue which were positive for EAP, mAAP, and gamma-GTP, but negative for DP IV. Using antibodies against and cDNA for DP IV.
Contrary to these findings, the present invention shows that the adminisitration of DP IV inhibitors like isoleucyl thiazolidine produces an anxiolytic effect in rats. Surprisingly, the present invention shows, that the adminisitration of the DP IV inhibitor isoleucyl thiazolidine exhibits an anxiolytic effect in rats.

As a consequence of the resulting enhanced stability of the endogenous NPY (1-36) caused by the administration of DP IV inhibitors, NPY activity is maintained, increased or prolonged resulting in functionally active NPY Y1 receptor activity facilitating anxiolytic effects (see above).

The method of the present invention for treating anxiety in an animal, including humans, in need thereof, comprises potentiating NPY's presence by administrating inhibitors for DP IV, or related enzyme activities. Oral administration of a DP IV inhibitor may be preferable in most circumstances. By the administration of an inhibitor of DP IV or DP IV like enzyme activity, the half-life of the active form of NPY will be appreciably extended, increased or maintained under physiological conditions. The extended presence of active NPY will enhance the NPY Y1 receptor activity.

Also disclosed are pharmaceutical compositions. Such compositions comprise a therapeutically (or prophylactically) effective amount of the inhibitor (and/or a sugar pill to accompany administration of a DP IV inhibitor), and a pharmaceutically acceptable carrier or excipient, especially adapted for targeting the brain. Suitable carriers include but are not limited to saline, buffered saline, dextrose, water, glycerol, ethanol, and combinations thereof. The carrier and composition are preferably produced under good laboratory practices conditions and most preferably are sterile. The formulation is ideally selected to suit the mode of administration, in accordance with conventional practice.

Suitable pharmaceutically acceptable carriers include but are not limited to water, salt solutions (for example, NaCl), alcohols, gum arabic, vegetable oils, benzyl alcohols, polyethylene glycols, gelatin, carbohydrates such as lactose, amylose or starch, magnesium stearate, talc, viscous paraffin, perfume oil, fatty acid esters, hydroxymethylcellulose, polyvinyl pyrrolidone, etc. The pharmaceutical preparations can be sterilized and if desired mixed with auxiliary agents, for example, lubricants, preservatives, stabilizers, wetting agents, emulsifiers, salts for influencing osmotic pressure, buffers, coloring, flavoring and/or aromatic substances and the like which do not deleteriously react with the active compounds, but which improve stability, manufacturability and/or aesthetic appeal.

The compositions, if desired, can also contain minor amounts of wetting or emulsifying agents, or pH buffering agents. In addition, the composition can be a liquid solution, suspension, emulsion, tablet, pill, capsule, sustained release formulation, or powder. In addition, the composition can be formulated as a suppository, with traditional binders and carriers such as triglycerides. Oral formulations can include standard carriers such as pharmaceutical grades of mannitol, lactose, starch, magnesium stearate, polyvinyl pyrrolidone, sodium saccharine, cellulose, magnesium carbonate etc.

Further, the compositions can be formulated in accordance with routine procedures as a pharmaceutical composition adapted for intravenous administration to human beings. Typically, compositions for intravenous administration are solutions in sterile isotonic aqueous buffer. Where necessary, the composition may also include a solubilizing agent and a local anesthetic to ease pain at the site of the injection. Generally, the ingredients are supplied either separately or mixed together in unit dosage form, for example, as a dry lyophilized powder or water free concentrate in a hermetically sealed container such as an ampoule or sachette indicating the quantity of active compound. Where the composition is to be administered by infusion, it can be dispensed with an infusion bottle containing sterile pharmaceutical grade water, saline or dextrose/water. Where the composition is administered by injection, an ampoule of sterile water for injection or saline can be provided so that the ingredients may be mixed prior to administration.

Finally, compositions can be formulated as neutral or salt forms. Pharmaceutically acceptable salts include those formed with free amino groups such as those derived from hydrochloric, phosphoric, acetic, oxalic, tartaric acid, etc., and those derived from sodium, potassium, ammonium, calcium, ferric hydroxides, isopropylamine, triethylamine, 2-ethylamino ethanol, histidine, procaine, etc.

The amount of the composition which will be effective in the treatment of anxiety can be determined by standard clinical techniques. In addition, *in vitro* and/or *in vivo* assays may optionally be employed to help identify optimal dosage ranges. The precise dose to be employed in the formulation will also depend on the route of administration, and the seriousness of the disease or disorder, and should be decided according to the judgement of the practitioner and each patient's circumstances.

The present invention will now be illustrated with reference to the following examples focussing on the anxiolytic and stress-protective action in a genetic model of DP IV deficiency (example 1), the anxiolytic action of pharmacological doses of DP IV inhibitors within the CNS (example 2), the interaction and potentiation of NPY mediated anxiolytic effects (example 3), and the characterization of an anxiolytic mechanism based on potentiation of NPY Y1 receptor mediated effects (Example 4).

### EXAMPLES OF THE INVENTION

### Example 1

Spontaneous mutations of the DP IV gene observed in substrains of Fischer (F344) rats provide a model for studying the role of DP IV in behavioral regulation and adaptation to stress. The mutations in F344 rats result in a lack of DP IV-like enzymatic activity and are found in substrains from Germany (GER) and Japan (JAP) (Thompson et al., 1991; Tsuji et al., 1992) while rats from US (USA) and Hannover (HAN) breeders show significant enzyme activity. In F344JAP rats, a G633R substitution in the CD26 protein causes a greatly reduced expression of a mutant inactive enzyme (Tsuji et al., 1992; Cheng et al., 1999) while the other DP IV negative F344GER substrain expresses a non-active mutant enzyme (Thompson et al., 1991). The F344JAP rat is therefore be considered as an "protein knock-out" model (Cheng et al., 1999) while the F344GER substrain may represent a "protein over-expression" model (Shingu, Helfritz, Meyer, Schmidt, Mentlein, von Hörsten, submitted). On the basis of these findings, a direct comparison of mutant F344JAP and F344GER substrains with "wild-type" F344USA rats would allow the differentiation between the role of DP IV expression and activity on behavioral regulation and other neurological and psychophysiological functions *in vivo.* In the present example we report that DP IV deficient F344 substrains are less anxious and less responsive to stress-induced physiological changes.

*Animals*. F344USA, F344JAP and F344GER substrains were obtained from the different countries via Charles River Germany. F344Han rats, initially derived from the F344USA substrain, were obtained from a breeding colony at the Central Animal Laboratory at Hannover Medical School (for further information, see: http://www.mh-hannover.de/institut/tierlabor/f344.htm). All substrains were bred for one generation at the Central Animal Laboratory at Hannover and maintained in a specificpathogen-free facility at 25°C under a 12h light-12h dark cycle (light on at 0700 h), with ad libitum access to food and water. For the experiments age-matched weeks old F1 offspring of all substrains was used. The District Government, Hannover, Germany, approved all research and animal care procedures.

*Quantification of DP IV activity in tissue of F344 substrains.* Plasma, lung and various other tissue samples were kept frozen at -80°C until use. Tissue was homogenized and DP IV enzyme activity was detected by incubating the substrate, glycylproline p-nitroanilide (gly-Pro-pNA, 1mg/ml in PBS) (Bachem. Germany), and the color development was measured at 405nm.

*Social interaction (SI) test.* The SI test was carried out as first described by File (1980) and has initially been validated in the laboratory (Kask, Nguyen. Pabst, von Hörsten, submitted). Two rats, matched for genotype and body weight, were removed from home cages and exposed to the testing environment. The arena was a squared open field (50 x 50 x 50cm) made of aluminum, placed inside a sound isolation box (Coulbourn Instruments, Lehigh Valley, PA). For details of the apparatus see our previous study (von Hörsten et al., 1998c). The open field was lit by a red photo light bulb (Philips PF712E; 1.3 Lux). Rats were unfamiliar with the apparatus. Behavior was monitored using a video camera placed above the field inside the testing/isolation box. The SI behavior of both rats was recorded on-line from a monitor placed outside on top of the box. The following parameters were scored by an observer (HPN) unaware of the substrain of rats: duration of time spent in sniffing, following, crawling under and over other rats, but not passive body contact (resting, sleeping). An increase of the SI time is considered an anxiolytic-like response.

*Stress induced body weight loss.* On three consecutive days, age matched animals from Japanese (JAP), United States (USA) and German (GER) breeders were individually transported to a novel room and remained there for 1 h. On the first day a novel cage containing sawdust was used and animals placed in a standard animal rack. On the second day procedure was the same except that the cage was without sawdust. The stress procedure on the third was the same as on day 2 except that the cage was placed on the bottom of the novel room.

*Statistical analysis.* Data from repeated observations were analyzed by two-way analyses of variance for repeated measures (ANOVA) (factors: "substrain" and "change of body weight after stress" as repeated measure). Data obtained from simple measures such as DP IV activity or SI time were analyzed by one-way (factor: "substrain") ANOVA. Asterisks indicate significant post hoc effects vs. F344USA substrain (Control) obtained by Fisher's PLSD. All data are presented as means ± S.E.M.

*DP IV activity in F344 substrains.* Corresponding to the literature, F344GER and F344JAP substrains lack endogenous DP IV activity (Fig. 1). Thus, these rats provide a genetic model for the investigation of the physiological role of DP IV activity in behavioral regulation.

*Anxiety in the social interaction test:* Those F344 substrains that lack DP IV activity (F344JAP and F344GER) spent significantly more time in active social interaction with a novel arena (Fig. 2). Thus, the lack of endogenous DP IV-like activity mediates anxiolytic-like effects.

*Stress-induced body-weight loss:* F344JAP and F344GER lose significantly less body weight after repeated 1h transport stress. Thus, the lack of endogenous DP IV-like activity in these substrains reduces the physiological changes induced by moderate stress (Fig.3).

Together, these data demonstrate that in the genetic model of the DP IV deficient F344 rats the lack of endogenous DP IV activity cause awiolytic-like and stress-protective effects.

### Example 2

In the previous example we have demonstrated that anxiety and stress-responsiveness were reduced in a genetic model of DP IV deficiency. In the present example, we report that central administration (i.c.v.) of the DP IV inhibitor isoleucyl thiazolidine leads to anxiolytic-like effects in a well-established test of anxiety in rodents, the elevated plus maze test. We further report that also the emotionality of rats in response to novelty as measured by the open field paradigm (Denenberg et al., 1968) is less pronounced in isoleucyl thiazolidine treated rats without affecting activity.

*Animals.* Male WistarF/Han (WF) rats (Central Animal Laboratory, Hannover Medical School, Hannover, Germany, see http://www.mh-hannover.de/institut/tierlabor/wf.htm for details), weighing 350-390g, were housed in a sound-proof, temperature controlled (24.0±0.5°C) room under specific pathogen free conditions with a 12/12h dark/light cycle (lights on at 07.00 with illumination level of 80 Lux). Food (Altromin lab chow pellets) and tap water were available *ad libitum.* Under ketamine/xylasine (100/5 mg/kg, i.p.) anesthesia, the rats were fixed in a Kopf stereotaxic frame and implanted with cannulae (Plastic One, Inc., Roanoke, VA, USA) above the lateral ventricle. All research and animal care procedures were approved by the Lower Saxony district government (Hannover, Germany) and followed principles described in the European Community's Council Directive of 24 November 1986 (86/609/EEC).

*Surgery and i.c.v. application.* For surgery, rats were anesthetized and prepared with i.c.v. cannulaes (coordinates: A:-0.7mm caudal, L:1.4mm lateral to bregma; and V:3.2mm ventral to the skull surface; tooth bar +3.0 above ear bars) using standard stereotaxic procedures as described in detail elsewhere (von Hörsten et al., 1998a,b,c). After a 7-day recovery period, successful ventricle cannulation was confirmed by an angiotensin drinking response (von Hörsten et al., 1998a). Rats showing a positive drinking response (*n*=40) were then habituated to experimental handling by daily sham injections for seven days.

Animals were randomly divided into four experimental groups (n=8-10/group), which completed different behavioral tests of experimental anxiety. Animals in each group were treated in an identical way in each phase, receiving i.c.v. injections -60mins before behavioral testing: Artificial cerebrospinal fluid (aCSF) (Control), isoleucyl thiazolidine (0.05nmol), isoleucyl thiazolidine (5nmol) and isoleucyl thiazolidine (500nmol), isoleucyl thiazolidine was adjusted to the final concentration using buffered aCSF and applied in a volume of 5pl/min into the right lateral ventricle. The cannula was attached to a Hamilton microsyringe with approximately 30cm of polyethylene tubing and all compounds were infused at a rate of 5.0 µl/min using a TSE multichannel infusion pump (Bad Homburg, Germany).

*Response to novelty (Open field test).* Differences in the response to novelty induced by central DP IV antagonism were studied using an open field (OF) test. The general procedure has been described elsewhere in detail (von Hörsten et al., 1993, 1998d). However, the following modifications were applied: During the dark phase, rats were placed in a squared 50x50cm OF within a sound isolated box illuminated by red photo light. Spontaneous activity during a single continuous test session of 15min was recorded using a video path analyzer system (E61-21 Video Path Analyser system, Coulbourn instruments, PA, U.S.A.). The analyzer system determines behavior at 15 one min intervals, analyzing 14 data elements: Wall-, corner-, quadrant 1-4-, locomotion-, rest-, and rearing-time (all in sec.), stereo-, rearing-, rotation clockwise- and counterclockwise-events (all integers), and distance traveled (cm). Furthermore, the total incidence of fecal boli was counted after each session and the incidence and duration (sec) of grooming behavior was simultaneously recorded from a video monitor by a person blind to the treatment of the animals (von Hörsten et al., 1998c).

*Elevated plus maze (EPM) testing.* The elevated plus maze apparatus and the test procedure were adapted according to Fernandes and File 1996 based on general considerations and validation for use with rats. The E+ apparatus (TSE Systems, Bad Homburg, Germany) was made of gray plastic and had two open arms (50 x 10cm) and two enclosed arms of the same size with walls 40cm high, elevated 50cm above the ground. The maze was equipped with light beam sensors that enabled computerized measurement of E+ performance. The maze was lit with red light bulb (Philips PF712E; 1.5 Lux) placed 30 cm above maze in a way that closed arms remained in the shadow. At start of experiment the rat was placed on central platform (10 x 10cm), with its head facing the closed arm, and allowed to freely explore the maze for 5min. The following parameters were calculated: Total numbers of arm entries (TA); entries to closed arms (CA); entries to open arms (OA); percentage frequency of entries to open arms (%OA: OAx 100/TA); total trial duration (TT): 300s; duration of stay in closed arms (closed time; CLT); percentage share of CLT in total arms-stay duration (CLT×100/AT); duration of stay in open arms (open time; OT) and percentage share of OT in total arm-stay duration (%OT: OT×100/AT). In addition to the standard spatiotemporal measurements, "time spent and percentage of time on the central square" were recorded (center time, CT: duration of stay on platform in seconds; percentage share of CT in trial duration, %CT: CTx 100/TT). An increase of the time spent on the open arms is interpreted as an anxiolytic response, a decrease of this parameter an anxiogenic response, whereas the number of entries into closed arms provides an indication of general activity (Pellow et al., 1985).

*Statistical analysis*. For statistical analysis, behavioral raw data from every test minute were analyzed by two-way analyses of variance (ANOVA) for repeated measures (factors: treatment and time as a repeated measurement). Data obtained as totals from a session were analyzed by one-way (factor: treatment) ANOVA. Significant post hoc effects vs. aCSF (Control) obtained by Fisher's PLSD are indicated by an asterisk. All data are presented as means ± S.E.M.

*Response to isoleucyl thiazolidine in the open field:* isoleucyl thiazolidine had no effect on activity in the open field in a wide range of dosages (Fig. 4). As on characteristic of reduced emotionality in the open field, there was a dose-dependent reduction of the time spent close to the wall induced by isoleucyl thiazolidine (Fig.5). Very low dosage of 50pmol was already effective.

*Response to isoleucyl thiazolidine in the EPM:* isoleucyl thiazolidine at low dose (50pmol) increased the percentage of time spent on the open arms of the maze being indicative for an anxiolytic-like effect (Fig. 6). Together, these data indicate for the first time that the administration of pharmacological doses of the DP IV inhibitor isoleucyl thiazolidine produces dose dependent anxiolytic-like effects in two animal models of anxiety.

### Example 3

In the present example, we report that central administration (i.c.v.) of the DP IV inhibitor isoleucyl thiazolidine itself has also dose-dependent anxiolytic effects in the social interaction test of anxiety. We also report that the magnitude of anxiolysis by isoleucyl thiazolidine is similar to that produced by NPY. In addition, we show that combined application of isoleucyl thiazolidine and NPY has additive anxiolytic effects in the SI test. Finally, we show that 1 h and 12h food intake is less affected by isoleucyl thiazolidine treatment alone and in combinations.

*Animals*. Male WistarF/Han (WF) rats (Central Animal Laboratory, Hannover Medical School, Hannover, Germany, see http://www.mh-hannover.de/institut/tierlaborlwf.htm for details), weighing 330-370g, were housed in a sound-proof, temperature controlled (24.0±0.5°C) room under specific pathogen free conditions with a 12/12h dark/light cycle (lights on at 07.00 with illumination level of 80 Lux). Food (Altromin lab chow pellets) and tap water were available *ad libitum.* Under ketamine/xylasine (100/5 mg/kg, i.p.) anesthesia, the rats were fixed in a Kopf stereotaxic frame and implanted with cannulae (Plastic One, Inc., Roanoke. VA, USA) above the lateral ventricle. All research and animal care procedures were approved by the Lower Saxony district government (Hannover, Germany) and followed principles described in the European Community's Council Directive of 24 November 1986 (86/609/EEC).

*Surgery and i.c.v. application.* For surgery, rats were anesthetized and prepared with i.c.v. cannulae (coordinates: A:-0.7mm caudal, L: 1.4mm lateral to bregma; and V3.2mm ventral to the skull surface; tooth bar +3.0 above ear bars) using standard stereotaxic procedures as described in detail elsewhere (von Hörsten et al., 1998a,b,c). After a 7-day recovery period, successful ventricle cannulation was confirmed by an angiotensin drinking response (von Hörsten et al., 1998a). Rats showing a positive drinking response (*n*=59) were then habituated to experimental handling by daily sham injections for seven days.

Animals were randomly divided into two sets of four experimental groups (n=5-6/group), which completed different consecutive SI test of anxiety. Animals in each group were treated in an identical way in each phase, receiving i.c.v. injections -60mins and -45min before behavioral testing with different dosages (isoleucyl thiazolidine: 5pmol-500nmol; NPY: 50pmol-1.6nmol), isoleucyl thiazolidine was adjusted to the final concentration using buffered aCSF and applied in a volume of 5µl/min into the right lateral ventricle. The cannula was attached to a Hamilton microsyringe with approximately 30cm of polyethylene tubing and all compounds were infused in a total volume of 5.0µl at a rate of 5.0 µl/min using a TSE multichannel infusion pump (Bad Homburg, Germany). The time spent in active social interaction (SI-time), 1h and 12h food-intake were measured. Animals were repeatedly tested with randomly chosen treatment and always-new interaction partners. Tests were separated with at least 4 days from each other and always conducted in the dark cycle. Five tests series were performed. Each test, spanning four groups (aCSF+aCSF; aCSF+NPY; isoleucyl thiazolidine+aCSF; isoleucyl thiazolidine+NPY) of 5-6 animals per condition.

*Social interaction (SI) test.* The SI test was carried out as first described by File (1980) and has initially been validated in the laboratory (Kask, Nguyen, Pabst, von Hörsten, submitted). Two rats, matched for genotype and body weight, were removed from home cages and exposed to the testing environment. The arena was a squared open field (50 x 50 x 50cm) made of aluminum, placed inside a sound isolation box (Coulbourn Instruments, Lehigh Valley, PA). For details of the apparatus see our previous study (von Hörsten et al., 1998c). The open field was lit by a red photo light bulb (Philips PF712E; 1.3 Lux). Rats were unfamiliar with the apparatus. Behavior was monitored using a video camera placed above the field inside the testing/isolation box. The SI behavior of both rats was recorded on-line from a monitor placed outside on top of the box. The following parameters were scored by an observer (HPN) unaware of the substrain of rats: duration of time spent in sniffing, following, crawling under and over other rats, but not passive body contact (resting, sleeping). An increase of the SI time is considered an anxiolytic-like response.

*Statistical analysis.* Data from repeated observations (food intake) were analyzed by two-way analyses of variance for repeated measures (ANOVA) (factors: "substrain" and "food intake" as repeated measure). Data obtained from simple measures such as DP IV activity or SI time were analyzed by one-way (factor: "substrain") ANOVA. Asterisks indicate significant post hoc effects vs. aCSF+aCSF (Control) obtained by Fisher's PLSD. All data are presented as means ± S.E.M.

*Dose dependent anxiolysis in the SI test by isoleucyl thiazolidine*. Central administration of the DP IV inhibitor isoleucyl thiazolidine (group: aCSF+isoleucyl thiazolidine: 5pmol-500nmol) produced "bell-shaped" dose-dependent anxiolytic-like effects in the social interaction test of anxiety (Fig. 7). This demonstrates that isoleucyl thiazolidine acts also in the SI test as a potent anxiolytic-like compound - similar to the EPM and the open field tests (see example 2). I.c.v. application of NPY (0.05-1.6nmol) had a similar anxiolytic-like effect (Fig. 7). This finding replicates the known anxiolytic-like action of NPY, as described in the background art. The comparison of isoleucyl thiazolidine-mediated effects with those of NPY indicates that the inhibitor is of similar potency. Interestingly, pretreatment of isoleucyl thiazolidine followed by NPY produced an additive effect over a wide range of dosages (Fig. 7), suggesting that these compounds act through the same mechanism. As described in the prior art, this mechanisms is most probably the activation of CNS Y 1 receptors.

*Minor effects of isoleucyl thiazolidine on feeding.* Fig.8 and Fig. 9 demonstrate that at 1 h NPY produced an "u-shaped" dose-dependent feeding effect (Fig. 8). isoleucyl thiazolidine produced a mild feeding effect that only at 0.05nmol dose reach significance (Fig.8). Combined treatment of isoleucyl thiazolidine followed by NPY differed not from NPY alone (except at highest non-physiological dosages), suggesting that the feeding effect of NPY is not mediated through a mechanism that is affected by isoleucyl thiazolidine. Most data in the prior art indicate that the dark cycle feeding effect of NPY is primarily Y5 receptor mediated. Over-night food-intake was not affected by any treatment, except at extremely high dosages (Fig. 9). Thus, central application of the DP IV inhibitor isoleucyl thiazolidine does not affect major feeding regulatory systems (i.e. the NPY Y5 receptor).

### Example 4

In the present example we report on the mechanism for the potentiation of NPY mediated anxiolytic-like effects at moderate dosages (isoleucyl thiazolidine, 50nmol; NPY, 0.8nmol) as shown in Fig. 7 of Example 3. We confirm that pre-treatment using the NPY Y1 receptor antagonist BIBP3226 can block the isoleucyl thiazolidine-induced potentiation of the NPY mediated anxiolytic-like effect on SI behavior and furthermore show that 1h feeding effects are only partly affected by blockade of the Y1R.

*Animals.* Male WistarF/Han (WF) rats (Central Animal Laboratory. Hannover Medical School, Hannover, Germany, see http://www.mh-hannover.de/institut/tierlabor/wf.htm for details), weighing 330±31 g±SD, were housed in a sound-proof, temperature controlled (24.0±0.5°C) room under specific pathogen free conditions with a 12/12h dark/light cycle (lights on at 07.00 with illumination level of 80 Lux). Food (Altromin lab chow pellets) and tap water were available *ad libitum.* Under ketamine/xylasine (100/5 mg/kg, i.p.) anesthesia, the rats were fixed in a Kopf stereotaxic frame and implanted with cannulae (Plastic One, Inc., Roanoke, VA, USA) above the lateral ventricle. All research and animal care procedures were approved by the Lower Saxony district government (Hannover, Germany) and followed principles described in the European Community's Council Directive of 24 November 1986 (86/609/EEC).

*Surgery and i.c.v. application*. For surgery, rats were anesthetized and prepared with i.c.v. cannulae (coordinates: A:-0.7mm caudal, L:1.4mm lateral to bregma; and V3.2mm ventral to the skull surface; tooth bar +3.0 above ear bars) using standard stereotaxic procedures as described in detail elsewhere (von Hörsten et al., 1998a,b,c). After a 7-day recovery period, successful ventricle cannulation was confirmed by an angiotensin drinking response (von Hörsten et al., 1998a). Rats showing a positive drinking response (*n*=56) were then habituated to experimental handling by daily sham injections for seven days.

Animals were randomly divided into eight experimental groups (n=6-8/group), which completed one SI test of anxiety: (1) aCSF+aCSF+aCSF; (2) BIBP+aCSF+aCSF; (3) aCSF+isoleucyl thiazolidine+aCSF; (4) BIBP+isoleucyl thiazolidine+aCSF; (5) aCSF+aCSF+NPY; (6) BIBP+aCSF+NPY; (7) aCSF+P32/89+NPY; (8) BIBP+isoleucyl thiazolidine+NPY. Animals in each group were treated in an identical way in each phase, receiving i.c.v. injections -60mins and -55min before behavioral testing. Experiments spanned two nights with groups counterbalances on both dark cycles. The injection cannula was attached to a Hamilton microsyringe with approximately 30cm of polyethylene tubing and all compounds were infused in a total volume of 5.0µl at a rate of 5.0 µl/min using a TSE multichannel infusion pump (Bad Homburg, Germany). The time spent in active social interaction (SI-time), 1h, and 12h food-intake were measured.

*Peptides and Antagonist.* Rat neuropeptide Y₁₋₃₆ was obtained from Polypeptide Laboratories (Wolfenbüttel, Germany). The NPY Y1 receptor antagonist BIBP3226 was purchased from American Peptide Company, Sunnyvale, CA, USA (Cat#:60-1-22B). All drugs were dissolved in sterile water and final dilutions were made with aCSF.

*Social interaction (SI) test.* The SI test was carried out as first described by File (1980) and has initially been validated in the laboratory (Kask, Nguyen, Pabst, von Hörsten, submitted). Two rats, matched for treatment, were removed from home cages and exposed to the testing environment. The arena was a squared open field (50 x 50 x 50cm) made of aluminum, placed inside a sound isolation box (Coulbourn Instruments. Lehigh Valley, PA). For details of the apparatus see our previous study (von Hörsten et al., 1998c). The open field was lit by a red photo light bulb (Philips PF712E; 1.3 Lux). Rats were unfamiliar with the apparatus. Behavior was monitored using a video camera placed above the field inside the testing/isolation box. The SI behavior of both rats was recorded on-line from a monitor placed outside on top of the box. The following parameters were scored by an observer (HPN) unaware of the substrain of rats: duration of time spent in sniffing, following, crawling under and over other rats, but not passive body contact (resting, sleeping). An increase of the SI time is considered an anxiolytic-like response.

*Statistical analysis.* Data obtained from the measures SI time and I h food intake were analyzed by one-way (factor: "treatment") ANOVA. In addition, three factorial (factors: "Antagonist", "Inhibitor", "NPY") ANOVA was performed to confirm general conclusions (data not shown). Asterisks indicate significant post hoc effects vs. aCSF+aCSF+aCSF (Control) while "#" signs indicate a significant difference of Y 1R antagonist treatment vs. the corresponding treatment without antagonist obtained by Fisher's PLSD. The level of significance in post hoc comparisons is indicated by asterisks with "*" = p<0.05; "**" = p<0.01; "***" = p<0.001) and by "#" symbols with "#" = p<0.05; "##" =p<0.01; "###" = p<0.001). All data are presented as means ± S.E.M.

*NPY Y1 receptor mediated potentiation of NPY-induced anxiolysis in the SI test by isoleucyl thiazolidine pre-treatment.* Combined central administration of isoleucyl thiazolidine at a dose of 50nmol and NPY at a dose of 0.8nmol dramatically potentiated the anxiolytic-like effect of NPY in the SI test (Fig. 10). This finding replicates at corresponding dosages the observation shown in Fig. 7. At this dose isoleucyl thiazolidine hemifumarate (p32/98) treatment alone acts not anxiolytic-like. However, the endogenous social investigatory behavior, the anxiolytic-like effect of NPY and the potentiated anxiolysis induced by combined isoleucyl thiazolidine+NPY treatment were all antagonized by Y1 receptor blockade. This indicates a tonic regulation of anxiety levels via the NPY Y1 receptor in the CNS and furthermore proofs that the anxiolytic-like effect of NPY as well as the potentiated anxiolysis induced by combined treatment all are primarily Y1 receptor mediated. Spontaneous feeding and NPY-induced feeding are only partly mediated by the Y1R and the non-significant slight increase of feeding induced by isoleucyl thiazolidine treatment is blocked by Y1R antagonisms (Fig. 11).

### REFERENCES

Aakerlund, L., U. Gether, T.W. Schwartz, and 0. Thastrup (1990), Yj receptors for neuropeptide Y are coupled to mobilization of intracellular calcium Agmo, A. and Belzung, C., Interactions between dopamine and GABA in the control of ambulatory activity and neophobia in the mouse, Pharmacol.Biochem.Behav., 59 (1998) 239-247.
Agmo, A. and Belzung, C., The role of subtypes of the opioid receptor in the anxiolytic action of chlordiazepoxide, Neuropharmacology, 37 (1998) 223-232.
Agmo, A., Galvan, A. , Heredia, A. and Morales, M., Naloxone blocks the antianxiety but not the motor effects of benzodiazepines and pentobarbital: experimental studies and literature review, Psychopharmacology Berl., 120 (1995) 186-194.
Arvat, E., Maccagno, B., Ramunni, J., Di-Vito, L., Gianotti, L., Broglio, F., Benso, A., Deghenghi, R., Camanni, F. and Ghigo, E., Effects of dexamethasone and alprazolam, a benzodiazepine, on the stimulatory effect of hexarelin, a synthetic GHRP, on ACTH, cortisol and GH secretion in humans, Neuroendocrinology., 67 (1998) 310-316.
Band, L. C., Pert, A., Williams, W., De Costa, B. R., Rice, K. C. and Weber, R. J., Central µ-opioid receptors mediate suppression of natural killer activity in vivo. Prog. Neuro. Endocrinol. Immunol., 5 (1992) 95-101.
Baram, T.Z., Yi, S., Avishai, E.S. and Schultz, L., Development neurobiology of the stress response: multilevel regulation of corticotropin-releasing hormone function, Ann.N.Y.Acad.Sci., 814 (1997) 252-265.
Baraniuk, J.N., Castellino, S., Lundgren, J.D., Goff, J., Mullol, J., Merida, M., Shelhamer, J,H. and Kaliner, M.A. (1990), Neuropeptide Y (NPY) in human nasal mucosa. J. Respir. Cell Mol. Biol. 3, 165-173;
Bard, J.A.,'Walker, M.W., Branchek, T.A. and Weinshank, R.L. (1995), J. Biol. Chem., 270, 26762-26765. Cloning and functional expression of Y4 subtype receptor for pancreatic polypeptide, neuropeptide Y, and peptide YY.
Berkenbosch, F., van-Oers, J., del-Rey, A., Tilders, F. and Besedovsky, H., Corticotropin-releasing factor-producing neurons in the rat activated by interleukin-1, Science, 238 (1987) 524-526.
Bernstein, HG.. Schön, E., Ansorge, S., Röse, I. Dorn, A. Immunolocalization of dipeptidyl aminopeptidase (DAP IV) in the developing human brain, Int J Dev Neurosci, 55 (1987) 237-42
Bertolucci, M., Perego, C. and De Simoni, M. G., Central opiate modulation of peripheral IL-6 in rats, NeuroReport, 7 (1996) 1181-1184.
Bertolucci, M., Perego, C. and de Simoni, M. G., Interleukin-6 is differently modulated by central opioid receptor subtypes, Am. J. Physiol., 273 (1997) R956-R959.
Bileviciute, I., Stenfors, C., Theodorsson, E., Beckman, M. and Lundeberg, T., Significant changes in neuropeptide concentrations in the brain of normotensive (WKY) and spontaneously hypertensive (SHR) rats following knee joint monoarthritis, Brain Res., 704 (1995) 71-78.
Britton, D.R., Koob, G.F., Rivier, J. and Vale, W., Intraventricular corticotropin-releasing factor enhances behavioral effects of novelty, Life Sci., 31 (1982) 363-367.
Britton, K.T., Lee, G. and Koob, G.F., Corticotropin releasing factor and amphetamine exaggerate partial agonist properties of benzodiazepine antagonist Ro 15-1788 in the conflict test, Psychopharmacology Berl., 94 (1988) 306-311.
Brown, M.R., Fisher, L.A., Spiess, J., Rivier, J., Rivier, C. and Vale, W., Comparison of the biologic actions of corticotropin-releasing factor and sauvagine, Regul.Pept., 4 (1982) 107-114.
Calenco, C.G., Dauge, V., Gacel, G., Feger, J. and Roques, B.P., Opioid delta agonists and endogenous enkephalins induce different emotional reactivity than mu agonists after injection in the rat ventral tegmental area. Psychopharmacology Berl., 103 (1991) 493-502.
Carr. D.J., Rogers, T.J. and Weber, R.J., The relevance of opioids and opioid receptors on immunocompetence and immune homeostasis. Proc.Soc.Exp.Biol.Med., 213 (1996) 248-257.
Chen, C., Dagnino, R., De-Souza, E.B., Grigoriadis, D.E., Huang, C.Q., Kim, K.I., Liu, Z., Moran, T., Webb, T.R., Whitten, J.P., Xie, Y.F. and McCarthy, J.R., Design and synthesis of a series of non-peptide high-affinity human corticotropin-releasing factor1 receptor antagonists, J.Med.Chem., 39 (1996) 4358-4360.
Chronwall, B.M., Anatomy and physiology of the neuroendocrine arcuate nucleus, Peptides, 6 Suppl 2 (1985) 1-11.
Colmers, W. and Wahlestedt, C. (1993) The biology of neuropeptide Y and related peptides. Humana Press, Totowa, New Jersey.
Conte, D.B., Rey, M., Boudouresque, F., Giraud, P., Castanas, E., Millet, Y., Codaccioni, J.L. and Oliver, C., Effect of 41-CRF antiserum on the secretion of ACTH, B-endorphin and alpha-MSH in the rat, Peptides, 4 (1983) 301-304.
Daniels, A.J., Matthews, J.E., Slepetis, R.J., Jansen, M., Viveros, O.H., Tadepalli, A., Harrington, W., Heyer, D., Landavazo, A., Leban, J.J. and et, a., High-affinity neuropeptide Y receptor antagonists, Proc.Natl.Acad.Sci.U.S.A., 92 (1995) 9067-9071.
De Bault, Le., Mitro, A., Enzyme histochemical studies of membrane proteases in rat subfornical organ, Acta Histochem, 96 (1994) 355-64
Devine, D.P., Taylor, L., Reinscheid, R.K., Monsma-FJ, J., Civelli, O. and Akil, H., Rats rapidly develop tolerance to the locomotor-inhibiting effects of the novel neuropeptide orphanin FQ, Neurochem.Res., 21 (1996) 1387-1396.
Dieterich, K.D., Lehnert, H. and De-Souza, E.B., Corticotropin-releasing factor receptors: an overview, Exp.Clin.Endocrinol.Diabetes, 105 (1997) 65-82.
Doods, H.N., Wieland, H.A., Engel, W., Eberlein, W., Willim, K.D., Entzeroth, M., Wienen, W. and Rudolf, K., BIBP 3226, the first selective neuropeptide Y 1 receptor antagonist: a review of its pharmacological properties, Regul.Pept., 65 (1996) 71-77.
Dubový, P., Dipeptidylpeptidase IV activity in Meissner corpuscles of rhesus monkey and is possible function, Brain Res, 461 (1988) 186-9
Duke-Cohan JS, Morimoto C, Rocker JA, Schlossman SF: Serum high molecular weight dipeptidyl peptidase IV (CD26) is similar to a novel antigen DPPT-L released from activated T cells, The journal of immunology 156, 1714-21 (1996)
Duke-Cohan JS, Tang W, Schlossman SF: Attractin: A cub-family protease involved in T cell-monocyte/macrophage interactions, Adv. Exp. Med. Biol. 477, 173-185 (2000)
Dumont, Y., Fournier, A., St, P. S. and Quirion, R., Autoradiographic distribution of [125I]Leu31,Pro34]PYY and [12SI]PYY3-36 binding sites in the rat brain evaluated with two newly developed Y1 and Y2 receptor radioligands, Synapse, 22 (1996) 139-158.
Dunn, A.J., Berridge, C.W., Lai, Y.I. and Yachabach, T.L., CRF-induced excessive grooming behavior in rats and mice, Peptides, 8 (1987) 841-844.
Egawa, M., Yoshimatsu, H. and Bray, G.A., Neuropeptide Y suppresses sympathetic activity to interscapular brown adipose tissue in rats, Am.J.Physiol., 260 (1991) R328-R334
Eghbal, A.M., Hatalski, C.G., Avishai, E.S. and Baram, T.Z., Corticotropin releasing factor receptor type II (CRF2) messenger ribonucleic acid levels in the hypothalamic ventromedial nucleus of the infant rat are reduced by maternal deprivation, Endocrinology, 138 (1997) 5048-5051.
Ekrivan, R., Servenius, B., Castro, M.G., Lowry, P.J., Cederlund, A.S., Bergman, O. and Sjogren, H.O., Biosynthesis of corticotropin-releasing hormone in human T-lymphocytes, J.Neuroimmunol., 44 (1993) 7-13.
File, S.E., The contribution of behavioural studies to the neuropharmacology of anxiety, Neuropharmacology, 26 (1987) 877-886.
Florin, S., Suaudeau, C., Meunier, J.C. and Costentin, J., Nociceptin stimulates locomotion and exploratory behaviour in mice. Eur.J.Pharmacol., 317 (1996) 9-13.
Fuhlendorff, J., U. Gether, L. Aakerlund, N. Langeland-Johansen, H. Togerson, S.GF. Melberg, U.B. Olsen, 0. Thastrup, and T.W. Schwartz 31 34 (1990), [LeU , Pro I Neuropeptide Y: A specific Yj receptor agonist, Proc. Natl. Acad. Sci. 87, 182-186.
Gaveriaux, R.C., Matthes, H.W., Peluso, J. and Kieffer, B.L., Abolition of morphine-immunosuppression in mice lacking the mu-opioid receptor gene, Proc.Natl.Acad.Sci. U.S.A., 95 (1998) 6326-6330.
Gehlert. D.R., Multiple receptors for the pancreatic polypeptide (PP-fold) family: physiological implications, Proc.Soc.Exp.Biol.Med., 218 (1998) 7-22.
Gerald, C., Walker, M,W., Criscione, L., Gustafson, E.L., Batzi-Hartmann, C., Smith, K.E" Vaysse, P., Durkin, M.M., Laz. T.M., Linemeyer, D.L., Schaffhauser, A.O., Whitebread, S., Hofbauer, K.G., Taber, R.I., Branchek. T,A. and Weinshank, R.L. (1996). A receptor subtype involved in neuropeptide-Y-induced food intake. Nature, 382, 168-171.
Gerald, C., Walker, M.W., Vaysse, P.J.-J, He, C., Branchek, T.A. and Weinshank, R.L. (1995) J. Biol. Chem., 270, 26758-26761. Expression cloning and pharmacological characterisation of a human hippocampal neuropeptide Y/peptide YY Y2 receptor subtype.
Gosnell, B.A., Levine, A.S. and Morley, J.E., The stimulation of food intake by selective agonists of mu, kappa and delta opioid receptors, Life Sci., 38 (1986) 1081-1088.
Grosskreutz, C.L. and Brody, M.J., Regional hemodynamic responses to central administration of corticotropin-releasing factor (CRF), Brain Res., 442 (1988) 363-367.
Grouzmann, E., Buclin, T., Martire, M., Cannizzaro, C., Dorner, B., Razaname, A. and Mutter, M., Characterization of a selective antagonist of neuropeptide Y at the Y2 receptor. Synthesis and pharmacological evaluation of a Y2 antagonist, J.Biol.Chem., 272 (1997) 7699-7706.
Grundemar, L., Grundstrom, N., joahansson, I.G.M., Andersson, R.G.G. and Hakanson, R. (1990) Suppression by neuropeptide Y of capsaicin- sensitive sensory nerve-mediated contraction in guinea-pig airways. Br. J. Pharmacol., 99, 473-476.
Grundemar, L., Wahlestedt, C. and Wang, Z.Y. (1993) Neuropeptide Y suppresses the neurogenic inflammatory response in the rabbit eye; mode of action. Regul. Pept., 43, 57-64.
Grunditz, T., Uddman, R. and Sundler, F. (1994) Origin and peptide content of nerve fibers in the nasal mucosa of rats. Anat. Embryol. 189, 327-337.
Gue, M., Junien, J.L., Reeve-JR, J., Rivier, J., Grandt, D. and Tache, Y., Reversal by NPY, PYY and 3-36 molecular forms of NPY and PYY of intracistemal CRF-induced inhibition of gastric acid secretion in rats, Br.J.Pharmacol., 118 (1996) 237-242.
Hartel-Schenk, S., Gossrau, R., Reutter,W., Comparative immunohistochemistry and histochemistry of dipeptidyl peptidase IV in rats organs during development, Histochem J, 22 (1990) 567-78
Heilig, M. and Murison, R., Intracerebroventricular neuropeptide Y suppresses open field and home cage activity in the rat, Regul.Pept., 19 (1987) 221-231.
Heilig, M., B. Söderpalm, J.A. Engel and E. Widerlöv, (1989), Centrally administered neuropeptide Y (NPY) produces anxiolytic-like effects in animal anxiety models, Psychopharmacology, 98, 524-529.
Heilig, M., McLeod, S., Brot, M., Heinrichs, S.C., Menzaghi, F., Koob, G.F. and Britton, K.T., Anxiolytic-like action of neuropeptide Y: mediation by Y 1 receptors in amygdala, and dissociation from food intake effects, Neuropsychopharmacology., 8 (1993) 357-363.
Heilig, M., Soderpalm, B., Engel, J.A. and Widerlov, E., Centrally administered neuropeptide Y (NPY) produces anxiolytic-like effects in animal anxiety models, Psychopharmacology Berl., 98 (1989) 524-529.
Heinrichs, S.C., Lapsansky, J., Behan, D.P., Chan, R.K. , Sawchenko, P.E., Lorang, M., Ling, N., Vale, W.W. and De-Souza, E.B., Corticotropin-releasing factor-binding protein ligand inhibitor blunts excessive weight gain in genetically obese Zucker rats and rats during nicotine withdrawal, Proc.Natl.Acad.Sci.U.S.A., 93 (1996) 15475-15480.
Heinrichs, S.C., Pich, E.M., Miczek, K.A., Britton, K.T. and Koob, G.F., Corticotropin-releasing factor antagonist reduces emotionality in socially defeated rats via direct neurotropic action, Brain Res., 581 (1992) 190-197.
Hcrz, A., Opioid reward mechanisms: a key role in drug abuse?. Can.J.Physiol.Pharmacol., 76 (1998) 252-258.
Herzog, H., Y.J. Hort. H.J. Ball. G. Hayes, J. Shine, and L.A. Selbie (1992), Cloned human neuropeptide Y receptor couples to two different second messenger systems, Proc. Natl. Acad. Sci. U.S.A. 89, 5794-5798.
Hoehe, M. and Duka, T., Opiates increase plasma catecholamines in humans, Psychoneuroendocrinology., 18 (1993) 141-148.
Hoffman, K.E., Maslonek, K.A., Dykstra, L.A. and Lysle, D.T., Effects of central administration of morphine on immune status in Lewis and Wistar rats. In B.M. Sharp, T.K. Eisenstein, J.J. Madden and H. Friedman (Eds.) The brain immune axis and substance abuse, Plenum Press, New York, 1995, pp. 155-159.
Hong, W., Petell, J.K., Swank, D., Sanford, J., Hixson, D.C., Doyle, D., Expression of dipetidyl peptidase IV in rat tissues is mainly regulated at the mRNA levels. Exp. Cell. Res. , 182 (1989), 256-266
Hörsten, S. v., Dimitrijevic M., Markovic B. M. and Jankovic B. D., Effect of early experience on behavior and immune response in the rat, Physiol Behav, 54 (1993) 931-40.
Hörsten, S.v., Ballof, J., Helfritz, F., Nave, H., Meyer, D., Schmidt, R.E., Stalp, M., Klemm, A., Tschemig, T. and Pabst, R., Modulation of innate immune functions by intracerebroventricularly applied Neuropeptide Y: Dose and time dependent effects, Life Sci., 63 (1998a) 909-922.
Hörsten, S.v., Exton M. S., Voge J., Schult M., Nagel E., Schmidt R. E., Westermann J. and Schedlowski M., Cyclosporine A affects open field behavior in DA rats, Pharmacol Biochem Behav, 60 (1998d) 71-6.
Hörsten, S.v., Exton, N.G., Exton, M.S., Helfritz, F., Nave, H., Ballof, J., Stalp, M. and Pabst, R., Brain NPY Y1 receptors rapidly mediate the behavioral response to novelty and a compartment-specific modulation of granulocyte function in blood and spleen, Brain Res., 806 (1998c) 282-286.
Hörsten. S.v., Nave, H., Ballof, J., Helfritz, F., Meyer, D., Schmidt, R.E., Stalp, M., Exton, N.G., Exton, M.S., Straub, R.H., Radulovic, J. and Pabst, R., Centrally applied NPY mimics immunoactivation induced by nonanalgesic doses of Met-Enkephalin, Neuroreport., 9 (1998b) 3881-3885.
Horvath, T.L., Naftolin, F., Kalra, S.P. and Leranth, C., Neuropeptide-Y innervation of beta-endorphin-containing cells in the rat mediobasal hypothalamus: a light and electron microscopic double immunostaining analysis [published erratum appears in Endocrinology 1996 Feb;137(2):532],
Endocrinology, 131 (1992) 2461-2467.
Jankovic, B.D. and Radulovic, J., Enkephalins, brain and immunity: modulation of immune responses by methionine-enkephalin injected into the cerebral cavity, Int.J.Neurosci., 67 (1992) 241-270.
Jenck, F., Moreau, J.L., Martin, J.R., Kilpatrick, G.J., Reinscheid, R.K., Monsma-FJ, J., Nothacker, H.P. and Civelli, O., Orphanin FQ acts as an anxiolytic to attenuate behavioral responses to stress, Proc.Natl.Acad.Sci.U.S.A., 94 (1997) 14854-14858.
Kalra, P.S., Norlin, M. and Kalra, S.P., Neuropeptide Y stimulates beta-endorphin release in the basal hypothalamus: role of gonadal steroids, Brain Res., 705 (1995) 353-356.
Karalis, K., Muglia, L.J., Bae, D., Hilderbrand, H. and Majzoub, J.A., CRH and the immune system, J.Neuroimmunol., 72 (1997) 131-136.
Kask, A., L. Rägo and J. Harro, (1998), NPY Y1 receptors in the dorsal periaqueductal gray matter regulate anxiety in the social interaction test, Neuroreport, 9, 2713-2716.
Kask. A., Rago. L. and Harro, J., Anxiolytic-like effect of neuropeptide Y (NPY) and NPY13-36 microinjected into vicinity of locus cocruleus in rats, Brain Res., 788 (1998) 345-348.
Kato, T., Hama, T., Nagatsu, T., Kuzuya, H., Sakakibara, S., Changes of X-prolyl dipeptidyl-aminopeptidase activity in developing rat brain, Experientia, 35 (1979) 1329-30
Kiritsy, R.J., Appel, N.M., Bobbitt, F.G. and Van-Loon, G.R., Effects of mu-opioid receptor stimulation in the hypothalamic paraventricular nucleus on basal and stress-induced catecholamine secretion and cardiovascular responses, J.Pharmacol.Exp.Ther., 239 (1986) 814-822.
Kiritsy, R.J., Marson, L. and Van-Loon, G.R., Sympathoadrenal, cardiovascular and blood gas responses to highly selective mu and delta opioid peptides, J.Pharmacol.Exp.Ther., 251 (1989) 1096-1103.
Konig. M., Zimmer, A.M., Steiner, H., Holmes, P.V., Crawley, J.N., Brownstein, M.J. and Zimmer, A., Pain responses, anxiety and aggression in mice deficient in pre-proenkephalin, Nature, 383 (1996) 535-538.
Koob. G.F. and Bloom. F.E., Corticotropin-releasing factor and behavior, Fed.Proc., 44 (1985) 259-263.
Kotz, C.M., Grace, M.K., Billington, C.J. and Levine, A.S., The effect of norbinaltorphimine, beta-funaltrexamine and naltrindole on NPY-induced feeding, Brain Res., 631 (1993) 325-328.
Krepela E, Kraml J, Vicar J, Kadlecova L, Kasafirek E: Dipeptidyl peptidase IV hydrolyses Gastric Inhibitory Polypeptide, Glucagon-like Peptide-1 (7-36)amide, Peptide Histidine Methionin and is Responsible for their Degradation in Human Serum, Eur. J. Biochem. 214, 829-835 (1993)
Lacroix, J.S., Ulman, L.G. and Potter, E.K. (1994) Modulation by neuropeptide Y of parasympathetic nerve-evoked nasal vasodilitation via Y2 prejunctional receptor. Br. J. Pharmacol., 113, 479-484.
Lambert, P.D., Wilding, J.P., al-Dokhayel, A.A., Gilbey, S.G. and Bloom, S.R., The effect of central blockade of kappa-opioid receptors on neuropeptide Y-induced feeding in the rat, Brain Res., 629 (1993) 146-148.
Leu, S.J. and Singh, V.K., Modulation of natural killer cell-mediated lysis by corticotropin-releasing neurohormone, J.Neuroimmunol., 33 (1991) 253-260.
Levine, A.S. and Billington, C.J., Opioids. Are they regulators of feeding?, Ann.N.Y.Acad.Sci., 575 (1989) 209-219.
Locke, K.W. and Holtzman, S.G., Behavioral effects of opioid peptides selective for mu or delta receptors. 1. Morphine-like discriminative stimulus effects, J.Pharmacol.Exp.Ther., 238 (1986) 990-996.
Loh, H.H., Liu, H.C., Cavalli, A., Yang, W., Chen, Y.F. and Wei, L.N., mu Opioid receptor knockout in mice: effects on ligand-induced analgesia and morphine lethality, Brain Res.Mol.Brain Res., 54 (1998) 321-326.
Lundberg, J. M., Pharmacology of cotransmission in the autonomic nervous system: Intergrative aspects on amines, neruopeptides, adenosine triphosphaste, amino acids and nitric oxide, Pharmacol.Rev., 48 (1996) 113-178.
Lundberg, J.M., Hensen, A., Larsson, 0., Rudehill, A., Saria, A & Fredholm B.B., (1988). Neuropeptide Y receptor in pig spleen; binding characteristics, reduction of cAMP formation and calcium antagonist inhibition of vasoconstriction. Eur. J. Pharmacol. Vol. 45; 21-29
Lysle, D.T., Hoffman, K.E. and Dykstra, L.A., Evidence for the involvement of the caudal region of the periaqueductal gray in a subset of morphine-induced alterations of immune status, J.Pharmacol.Exp.Ther., 277 (1996) 1533-1540.
Makino, S., Takemura, T., Asaba, K., Nishiyama, M., Takao, T. and Hashimoto, K., Differential regulation of type-1 and type-2alpha corticotropin-releasing hormone receptor mRNA in the hypothalamic paraventricular nucleus of the rat, Brain Res.Mol.Brain Res., 47 (1997) 170-176.
Mamiya, T., Noda, Y., Nishi, M., Takeshima, H. and Nabeshima, T., Enhancement of spatial attention in nociceptin/orphanin FQ receptor-knockout mice, Brain Res., 783 (1998) 236-240.
Manabe, T., Noda, Y., Mamiya, T., Katagiri, H., Houtani, T., Nishi, M., Noda, T., Takahashi, T., Sugimoto, T., Nabeshima, T. and Takeshima, H., Facilitation of long-term potentiation and memory in mice lacking nociceptin receptors, Nature, 394 (1998) 577-581.
Matthes, H.W., Maldonado, R., Simonin, F., Valverde, O., Slowe, S., Kitchen, I., Befort, K., Dierich, A., Le-Meur, M., Dolle, P., Tzavara, E., Hanoune, J., Roques, B.P. and Kieffer, B.L., Loss of morphine-induced analgesia, reward effect and withdrawal symptoms in mice lacking the mu-opioid-receptor gene [see comments], Nature, 383 (1996) 819-823.
May, C.N., Dashwood, M.R., Whitehead, C.J. and Mathias, C.J., Differential cardiovascular and respiratory responses to central administration of selective opioid agonists in conscious rabbits: correlation with receptor distribution, Br.J.Pharmacol., 98 (1989) 903-913.
Mellado, M.L., Gibert, R.J., Chover, A.J. and Mico, J.A., Effect on nociception of intracerebroventricular administration of low doses of neuropeptide Y in mice, Life Sci., 58 (1996) 2409-2414.
Mellon. R.D. and Bayer, B.M., Evidence for central opioid receptors in the immunomodulatory effects of morphine: review of potential mechanism(s) of action, J.Neuroimmunol., 83 (1998) 19-28.
Mellon. R.D. and Bayer, B.M., Role of central opioid receptor subtypes in morphine-induced alterations in peripheral lymphocyte activity, Brain Res., 789 (1998) 56-67.
Menzaghi, F., Heinrichs, S.C., Pich, E.M., Tilders, F.J. and Koob, G.F., Functional impairment of hypothalamic corticotropin-releasing factor neurons with immunotargeted toxins enhances food intake induced by neuropeptide Y, Brain Res., 618 (1993) 76-82.
Menzaghi, F., Howard, R.L., Heinrichs, S.C., Vale, W., Rivier, J. and Koob, G.F., Characterization of a novel and potent corticotropin-releasing factor antagonist in rats, J.Pharmacol.Exp.Ther., 269 (1994)564-572.
Mercer, M.E. and Holder, M.D., Food cravings, endogenous opioid peptides, and food intake: a review, Appetite., 29 (1997) 325-352.
Meunier, J.C., Mollereau, C., Toll, L., Suaudeau, C., Moisand, C., Alvinerie, P., Butour, J.L., Guillemot, J.C., Ferrara, P., Monsarrat, B. and et, a., Isolation and structure of the endogenous agonist of opioid receptor-like ORL1 receptor [see comments], Nature, 377 (1995) 532-535.
Millan, M.J., Kappa-opioid receptors and analgesia, Trends.Pharmacol.Sci., 11 (1990) 70-76.
Minami, M. and Satoh, M., Molecular biology of the opioid receptors: structures, functions and distributions, Neurosci.Res., 23 (1995) 121-145.
Mitro, A., Lojda, Z., Histochemistry of proteases in ependyma choroid plexus and leptomeninges, Histochemistry, 88 (1988) 645-6
Mogil, J.S., Grisel, J.E., Reinscheid, R.K., Civelli, O., Belknap, J.K. and Grandy, D.K., Orphanin FQ is a functional anti-opioid peptide, Neuroscience, 75 (1996) 333-337.
Mollereau, C., Parmentier, M., Mailleux, P., Butour, J.L., Moisand, C., Chalon, P., Caput, D., Vassart, G. and Meunier, J.C., ORL1, a novel member of the opioid receptor family. Cloning, functional expression and localization, FEBS Lett., 341 (1994) 33-38.
Motta, V. and Brandao, M.L., Aversive and antiaversive effects of morphine in the dorsal periaqueductal gray of rats submitted to the elevated plus-maze test , Pharmacol.Biochem.Behav., 44 (1993) 119-125.
Nässel, Dr., Mentlein, R., Bollner, T., Karlsson, A., Proline-specific dipeptidyl peptidase activity in the cockroach brain and intestine: partial characterization, distribution, and inactivation of tachykinin-related peptides, J Comp Neurol, 418 (2000) 81-92
Nishi, M., Houtani, T., Noda, Y., Mamiya, T., Sato, K., Doi, T., Kuno, J., Takeshima, H., Nukada, T., Nabeshima, T., Yamashita, T., Noda, T. and Sugimoto, T., Unrestrained nociceptive response and disregulation of hearing ability in mice lacking the nociceptin/orphaninFQ receptor, EMBO J., 16 (1997) 1858-1864.
Noble, F., Smadja, C., Valverde, O., Maldonado, R., Coric, P., Turcaud, S., Foumie, Z.M. and Roques, B.P., Pain-suppressive effects on various nociceptive stimuli (thermal, chemical, electrical and inflammatory) of the first orally active enkephalin-metabolizing enzyme inhibitor RB 120, Pain, 73 (1997) 383-391.
Noda, Y., Mamiya, T., Nabeshima, T., Nishi, M., Higashioka, M. and Takeshima, H., Loss of antinociception induced by naloxone benzoylhydrazone in nociceptin receptor-knockout mice, J.Biol.Chem., 273 (1998) 18047-18051.
Novak, J. E., Gomez-Flores, R., Calderon, S. N., Rice, K. C. and Weber, R. J., Rat natural killer cell, T cell and macrophage functions after intracerebroventricular injection of SNC80, J.Pharmacol.Exp.Ther., 286 (1998) 931-937.
O'Connor, B., O'Cuinn, G., Post-proline dipeptidyl-aminopeptidase from synaptosomal membranes of guinea-pig brain. A possible role for this activity in the hydrolysis of His-ProNH2, arising from the action of synaptosomal membrane pyroglutamate aminopeptidase on thyroliberin, Eur J Biochem, 154 (1986) 329-35
Olson, G.A., Olson, R.D. and Kastin, A.J., Endogenous opiates: 1995, Peptides, 17 (1996) 1421-1466.
Ono, N., Lumpkin, M.D., Samson, W.K., McDonald, J.K. and McCann, S.M., Intrahypothalamic action of corticotrophin-releasing factor (CRF) to inhibit growth hormone and LH release in the rat, Life Sci., 35 (1984) 1117-1123.
Pechnick, R.N., Effects of opioids on the hypothalamo-pituitary-adrenal axis, Annu.Rev.Pharmacol.Toxicol., 33 (1993) 353-382.
Pedrazzini, T., Seydoux, J., Kunstner, P., Aubert, J.F., Grouzmann, E., Beermann, F. and Brunner, H.R., Cardiovascular response, feeding behavior and locomotor activity in mice lacking the NPY Y 1 receptor [see comments]. Nat.Med., 4 (1998) 722-726.
Pfeiffer, A., Branti, V., Herz, A. and Emrich, H.M., Psychotomimesis mediated by kappa opiate receptors, Science, 233 (1986) 774-776.
Pomonis, J.D., Billington, C.J. and Levine, A.S., Orphanin FQ, agonist of orphan opioid receptor ORL1, stimulates feeding in rats, Neuroreport., 8 (1996) 369-371.
Potter, E.K. and M.J,D. McCloskey, (1992), [Leu 3', Leu 34 1 neuropeptide Y, a 45 selective functional agonist at neuropeptide Y receptors in anaesthetised rats, Neurosci. Lett. 134, 183-186,
Potter, EX, J. Fuhlendorff and T.W. Schwartz (1991), [Pro34 J neuropeptide Y selectively identifies postjunctional-mediated actions of neuropeptide Y in idvo in rats and dogs, Eur. J. Pharmacol. 193, 15-19.
Potter, EX, Mitchell, L., McCloskey, M.J., Tseng, A., Goodman, A.E., Shine, J. and McCloskey, D.I. (1989) Pre-and postjunctional actions of neuropeptide Y and related peptides. Regul. Pept. 25, 167-177.
Privette, T.H. and Terrian, D.M., Kappa opioid agonists produce anxiolytic-like behavior on the elevated plus-maze, Psychopharmacology Berl., 118 (1995) 444-450.
Radulovic, J. and Jankovic, B.D., Opposing activities of brain opioid receptors in the regulation of humoral and cell-mediated immune responses in the rat, Brain Res., 661 (1994) 189-195.
Radulovic, J., Miljevic, C., Djergovic, D., Vujic, V., Antic, J., von-Horsten, S. and Jankovic, B.D., Opioid receptor-mediated suppression of humoral immune response in vivo and in vitro: involvement of kappa opioid receptors, J.Neuroimmunol., 57 (1995) 55-62.
Rassnick, S., Heinrichs, S.C., Britton, K.T. and Koob, G.F., Microinjection of a corticotropin-releasing factor antagonist into the central nucleus of the amygdala reverses anxiogenic-like effects of ethanol withdrawal, Brain Res., 605 (1993) 25-32.
Reinscheid, R.K., Nothacker, H.P., Bourson, A., Ardati, A., Henningsen, R.A., Bunzow, J.R., Grandy, D.K., Langen, H., Monsma-FJ, J. and Civelli, O., Orphanin FQ: a neuropeptide that activates an opioidlike G protein-coupled receptor, Science, 270 (1995) 792-794.
Rioux, F., H. Bachelard, J.C. Martel and S. St.-Piere, (1986), The vasoconstrictor effect of neuropeptide Y and related peptides in the guinea pig isolated heart, Peptides, 7, 27-31.
Risdahl, J.M., Khanna, K.V., Peterson, P.K. and Molitor, T.W., Opiates and infection, J.Neuroimmunol., 83 (1998) 4-18.
Rivier, C., Rivier, J., Mormede, P. and Vale, W., Studies of the nature of the interaction between vasopressin and corticotropin-releasing factor on adrenocorticotropin release in the rat, Endocrinology. 115 (1984) 882-886.
Rivier, C.L. and Plotsky, P.M., Mediation by corticotropin releasing factor (CRF) of adenohypophysial hormone secretion, Annu.Rev.Physiol., 48 (1986) 475-494.
Rossi, G.C., Leventhal, L. and Pasternak, G.W., Naloxone sensitive orphanin FQ-induced analgesia in mice, Eur.J.Pharmacol., 311 (1996) R7-R8
Sandin, J., Georgieva, J., Schott, P.A., Ogren, S.O. and Terenius, L., Nociceptin/orphanin FQ microinjected into hippocampus impairs spatial learning in rats, Eur.J.Neurosci., 9 (1997) 194-197.
Saperstein, A., Brand, H., Audhya, T., Nabriski, D., Hutchinson, B., Rosenzweig, S. and Hollander, C.S., Interleukin 1 beta mediates stress-induced immunosuppression via corticotropin-releasing factor, Endocrinology, 130 (1992) 152-158.
Satoh, M. and Minami, M., Molecular pharmacology of the opioid receptors, Pharmacol.Ther., 68 (1995) 343-364.
Schwartz, T.W., J. Fuhlendorff, H, Langeland, J.C. T6gerson, S.P. Sheikh, (1989), in Neuropeptide Y - XIV Nobel Symposium, ed: V. Mutt; T. H6kfelt, K. Fuxe and J.M. Lundberg, Raven, N.Y. pp143.
Shavit, J. (1991) Stress-induced immune modulation in animals: opiates and endogenous opioid peptides. In: R. Ader, D.L. Felten and N. Cohen (Eds.), Psychoncuroimmunology, Vol. Academic Press, San Diego, pp. 789-804.
Shavit, Y., Depaulis. A., Martin, F.C., Terman, G.W., Pechnick, R.N., Zane, C.J., Gale, R.P. and Liebeskind, J.C., Involvement of brain opiate receptors in the immune-suppressive effect of morphine, Proc.Natl.Acad.Sci.U.S.A., 83 (1986) 7114-7117.
Sheikh, S.P., R. HAkanson and T.W. Schwartz, (1989), Yj and Y2receptors for neuropeptide Y, FEBS Lett. 245, 209-214.
Shippenberg, T.S., Bals, K.R. and Herz, A., Motivational properties of opioids: evidence that an activation of delta-receptors mediates reinforcement processes, Brain Res., 436 (1987) 234-239.
Simonin, F., Valverde, O., Smadja, C., Slowe, S., Kitchen, I., Dierich, A., Le-Meur, M., Roques, B.P., Maldonado, R. and Kieffer, B.L., Disruption of the kappa-opioid receptor gene in mice enhances sensitivity to chemical visceral pain, impairs pharmacological actions of the selective kappa-agonist U-50,488H and attenuates morphine withdrawal, EMBO J., 17 (1998) 886-897.
Sora, I., Funada, M. and Uhl, G.R., The mu-opioid receptor is necessary for [D-Pen2,D-Pen5]enkephalin-induced analgesia, Eur.J.Pharmacol., 324 (1997) R1-R2
Stanley, B.G. and Leibowitz, S.F., Neuropeptide Y injected in the paraventricular hypothalamus: a powerful stimulant of feeding behavior, Proc.Natl.Acad.Sci.U.S.A., 82 (1985) 3940-3943.
Stanley, B.G., Lanthier, D., Chin, A.S. and Leibowitz, S.F., Suppression of neuropeptide Y-elicited eating by adrenalectomy or hypophysectomy: reversal with corticosterone, Brain Res., 501 (1989) 32-36.
Stefano, G.B., Salzet, B. and Fricchione, G.L., Enkelytin and opioid peptide association in invertebrates and vertebrates: immune activation and pain, Immunol.Today, 19 (1998) 265-268.
Tatemoto, K., Carlquist, M. and Mutt, V., Neuropeptide Y--a novel brain peptide with structural similarities to peptide YY and pancreatic polypeptide, Nature, 296 (1982) 659-660.
Tatemoto, K., Neuropeptide Y: complete amino acid sequence of the brain peptide, Proc.Natl.Acad.Sci.U.S.A., 79 (1982) 5485-5489.
Tejedor, R.P., Costela, C. and Gibert, R.J., Neonatal handling reduces emotional reactivity and susceptibility to learned helplessness. Involvement of catecholaminergic systems, Life Sci., 62 (1998) 37-50.
Tian, M., Broxmeyer, H.E., Fan, Y., Lai, Z., Zhang, S., Aronica, S., Cooper, S., Bigsby, R.M., Steinmetz, R., Engle, S.J., Mestek, A., Pollock, J.D., Lehman, M.N., Jansen, H.T., Ying, M., Stambrook, P.J., Tischfield, J.A. and Yu, L., Altered hematopoiesis, behavior, and sexual function in mu opioid receptor-deficient mice, J.Exp.Med., 185 (1997) 1517-1522.
Tortella, F.C. and DeCoster, M.A., Kappa opioids: therapeutic considerations in epilepsy and CNS injury, Clin.Neuropharmacol., 17 (1994) 403-416.
Tsuda, M., Suzuki, T., Misawa, M. and Nagase, H., Involvement of the opioid system in the anxiolytic effect of diazepam in mice, Eur.J.Pharmacol., 307 (1996) 7-14.
Uehara, Y., Shimizu, H., Ohtani, K., Sato, N. and Mori, M., Hypothalamic corticotropin-releasing hormone is a mediator of the anorexigenic effect of leptin, Diabetes, 47 (1998) 890-893.
Vaughan, J., Donaldson, C., Bittencourt, J., Perrin, M.H., Lewis, K., Sutton, S., Chan, R., Turnbull, A.V., Lovejoy, D., Rivier, C. and et, a., Urocortin, a mammalian neuropeptide related to fish urotensin I and to corticotropin-releasing factor [see comments], Nature, 378 (1995) 287-292.
Wahlestedt, C. and Reis. D.J., Neuropeptide Y-related peptides and their receptors--are the receptors potential therapeutic drug targets?, Annu.Rev.Pharmacol.Toxicol., 33 (1993) 309-352.
Wahlestedt, C., N. Yanaihara and R. H. Akanson, (1986), Evidence for different pre- and post-junctional receptors for neuropeptide Y and related peptides, Regul. Pep. 13, 307-318.
Wahlestedt, C., Pich, E.M., Koob, G.F., Yee, F. and Heilig, M., Modulation of anxiety and neuropeptide Y-Y1 receptors by antisense oligodeoxynucleotides, Science, 259 (1993) 528-531.
Wettstein, J.G., Earley, B. and Junien, J.L., Central nervous system pharmacology of neuropeptide Y, Pharmacol. Ther., 65 (1995) 397-414.
Xu. X.J., Hao, J.X. and Wiesenfeld, H.Z., Nociceptin or antinociceptin: potent spinal antinociceptive effect of orphanin FQ/nociceptin in the rat, Neuroreport., 7 (1996) 2092-2094.
Zadina, J.E., Hackler, L., Ge, L.J. and Kastin, A.J., A potent and selective endogenous agonist for the mu-opiate receptor [see comments], Nature, 386 (1997) 499-502.
Zhao, X.J., Hoheisel, G., Schauer, J. and Bornstein, S.R., Corticotropin-releasing hormone-binding protein and its possible role in neuroendocrinological research. Horm.Metab.Res., 29 (1997) 373-378.
Zhu, Y. and Im, W. B., Block of sodium channel current by anticonvulsant U-54494A in mouse neuroblastoma cells, J.Pharmacol.Exp.Ther., 260 (1992) 110-116.

## Claims

1. Use of an inhibitor of dipeptidyl peptidase IV (DP IV or CD 26) for the production of a medicament for the treatment of anxiety.

2. The use according to claim 1, wherein the medicament reduces the degradation of the endogenous, CNS-localized neuropeptide Y (NPY).

3. The use according to any one of the preceding claims, **characterized in that** the inhibitors are used in combination with neuropeptide Y.

4. The use according to any one of the preceding claims, **characterized in that** the inhibitors are present in a physiologically compatible drug delivery vehicle.

5. The use according to any one of the preceding claims, wherein the inhibitor of dipeptidyl peptidase IV is selected from N-(N'-substituted glycyl)-2-cyanopyrrolidines, L-threo-isoleucyl thiazolidine, L-*allo-*isoleucyl thiazolidine, L-*threo*-isoleucyl pyrrolidine and L-*allo*-isoleucyl pyrrolidine.

6. The use according to any one of the preceding claims, wherein the inhibitor of dipeptidyl peptidase IV is L-threo-isoleucyl thiazolidine.

7. The use according to any one of the preceding claims, **characterized in that** the inhibitors are formulated as prodrugs of the general formula A-B-C, wherein A represents an amino acid, B represents a chemical bond between A and C or an amino acid, and C represents a stable or unstable inhibitor of dipeptidyl peptidase IV.

8. Use according to any one of the preceding claims, **characterized in that** the inhibitors are used parenterally, enterally, orally, by inhalation or as a suppository.

9. An inhibitor of dipeptidyl peptidase IV (DP IV or CD 26) for the treatment of anxiety.

10. The inhibitor of dipeptidyl peptidase IV of claim 9, **characterized in that** the degradation of the endogenous, CNS-localized neuropeptide Y (NPY) is reduced.

11. The inhibitor of dipeptidyl peptidase IV according to claim 9 or 10, **characterized in that** the inhibitor is used in combination with neuropeptide Y.

12. The inhibitor of dipeptidyl peptidase IV according to any one of claims 9 to 11, **characterized in that** the inhibitor is present in a physiologically compatible drug delivery vehicle.

13. The inhibitor of dipeptidyl peptidase IV according to any one of claims 9 to 12, **characterized in that** the inhibitor is selected from N-(N'-substituted glycyl)-2-cyanopyrrolidines, L-*threo*-isoleucyl thiazolidine, L-*allo*-isoleucyl thiazolidine, L-threo-isoleucyl pyrrolidine, and L-*allo*-isoleucyl pyrrolidine.

14. The inhibitor of dipeptidyl peptidase IV according to any one of claims 9 to 13, **characterized in that** the inhibitor is L-threo-isoleucyl thiazolidine.

15. The inhibitor of dipeptidyl peptidase IV according to any one of claims 9 to 14, **characterized in that** the inhibitor is formulated as prodrug of the general formula A-B-C, wherein A represents an amino acid, B represents a chemical bond between A and C or an amino acid, and C represents a stable or unstable inhibitor of dipeptidyl peptidase IV.

16. The inhibitor of dipeptidyl peptidase IV according to any one of claims 9 to 15, **characterized in that** the inhibitor is used parenterally, enterally, orally, by inhalation or as a suppository.

## Patentansprüche

1. Verwendung eines Inhibitors der Dipeptidyl Peptidase IV (DP IV oder CD26) für die Herstellung eines Medikaments zur Behandlung von Angst.

2. Die Verwendung nach Anspruch 1, wobei das Medikament den Abbau des endogenen, ZNS-lokalisierten Neuropeptids Y (NPY) reduziert.

3. Die Verwendung nach irgendeinem der vorstehenden Ansprüche, **dadurch** charakterisiert dass die Inhibitoren in Kombination mit Neuropeptid Y verwendet werden.

4. Die Verwendung nach irgendeinem der vorstehenden Ansprüche, **dadurch** charakterisiert dass die Inhibitoren in einem physiologisch verträglichen Medium für die Arzneimittelverabreichung vorliegen.

5. Die Verwendung nach irgendeinem der vorstehenden Ansprüche, wobei der Inhibitor der Dipeptidyl Peptidase IV ausgewählt ist aus N-(N'-substituierten Glycyl)-2-Cyanopyrrolidinen, L-*threo*-Isoleucyl Thiazolidin, L-*allo*-Isoleucyl Thiazolidin, L-*threo-*Isoleucyl Pyrrolidin und L-*allo*-Isoleucyl Pyrrolidin.

6. Die Verwendung nach irgendeinem der vorigen Ansprüche, wobei der Inhibitor der Dipeptidyl Peptidase IV L-*threo*-Isoleucyl Thiazolidin ist.

7. Die Verwendung nach irgendeinem der vorstehenden Ansprüche, **dadurch** charakterisiert dass die Inhibitoren als Prodrugs der allgemeinen Formel A-B-C formuliert sind, wobei A eine Aminosäure darstellt, B eine chemische Bindung zwischen A und C oder eine Aminosäure darstellt, und C einen stabilen oder instabilen Inhibitor der Dipeptidyl Peptidase IV darstellt.

8. Verwendung nach irgendeinem der vorstehenden Ansprüche, **dadurch** charakterisiert dass die Inhibitoren parenteral, enteral, oral, durch Inhalation oder als Zäpfchen verwendet werden.

9. Ein Inhibitor der Dipeptidyl Peptidase IV (DP IV oder CD26) zur Behandlung von Angst.

10. Der Dipeptidyl Peptidase IV Inhibitor nach Anspruch 9, **dadurch** charakterisiert dass der Abbau des endogenen, ZNS-lokalisierten Neuropeptids Y (NPY) reduziert ist.

11. Der Dipeptidyl Peptidase IV Inhibitor nach Anspruch 9 oder 10, **dadurch** charakterisiert dass der Inhibitor in Kombination mit Neuropeptid Y verwendet wird.

12. Der Dipeptidyl Peptidase IV Inhibitor nach irgendeinem der Ansprüche 9 bis 11, **dadurch** charakterisiert dass der Inhibitor in einem physiologisch verträglichen Medium für die Arzneimittelverabreichung vorliegt.

13. Der Dipeptidyl Peptidase IV Inhibitor nach irgendeinem der Ansprüche 9 bis 12, **dadurch** charakterisiert dass der Inhibitor aus N-(N'-substituierten Glycyl)-2-Cyanopyrrolidinen, L-*threo*-Isoleucyl Thiazolidin, L-*allo*-Isoleucyl Thiazolidin, L-*threo*-Isoleucyl Pyrrolidin und L-*allo*-Isoleucyl Pyrrolidin ausgewählt ist.

14. Der Dipeptidyl Peptidase IV Inhibitor nach irgendeinem der Ansprüche 9 bis 13, **dadurch** charakterisiert dass der Inhibitor L-*threo*-Isoleucyl Thiazolidin ist.

15. Der Dipeptidyl Peptidase IV Inhibitor nach irgendeinem der Ansprüche 9 bis 14, **dadurch** charakterisiert dass der Inhibitor als Prodrug der allgemeinen Formel A-B-C formuliert ist, wobei A eine Aminosäure darstellt, B eine chemische Bindung zwischen A und C oder eine Aminosäure darstellt, und C einen stabilen oder instabilen Inhibitor der Dipeptidyl Peptidase IV darstellt.

16. Der Dipeptidyl Peptidase IV Inhibitor nach irgendeinem der Ansprüche 9 bis 15, **dadurch** charakterisiert dass der Inhibitor parenteral, enteral, oral, durch Inhalation oder als Zäpfchen verwendet wird.

## Revendications

1. Utilisation d'un inhibiteur de la dipepdityl peptidase IV (DP IV ou CD 26) pour la production d'un médicament destiné au traitement de l'anxiété.

2. Utilisation selon la revendication 1, dans laquelle le médicament réduit la dégradation du neuropeptide Y (NPY) endogène localisé dans le CNS.

3. Utilisation selon l'une quelconque des revendications précédentes, **caractérisée en ce que** les inhibiteurs sont utilisés en combinaison avec le neuropeptide Y.

4. Utilisation selon l'une quelconque des revendications précédentes, **caractérisée en ce que** les inhibiteurs sont présents dans un véhicule de distribution de médicament physiologiquement compatible.

5. Utilisation selon l'une quelconque des revendications précédentes, dans laquelle l'inhibiteur de la dipeptidyl peptidase IV est choisi parmi des N-(N'-substitué glycyl)-2-cyanopyrrolidines, la L-*thréo*-isoleucyl thiazolidine, la L-*allo*-isoleucyl thiazolidine, la L-*thréo*-isoleucyl pyrrolidine et la L-*allo-*isoleucyl pyrrolidine.

6. Utilisation selon l'une quelconque des revendications précédentes, dans laquelle l'inhibiteur de la dipeptidyl peptidase IV est la L-*thréo*-isoleucyl thiazolidine.

7. Utilisation selon l'une quelconque des revendications précédentes, **caractérisée en ce que** les inhibiteurs sont formulés sous la forme de promédicaments de formule générale A-B-C, dans laquelle A représente un acide aminé, B représente une liaison chimique entre A et C ou un acide aminé, et C représente un inhibiteur stable ou instable de la dipepdityl peptidase IV.

8. Utilisation selon l'une quelconque des revendications précédentes, **caractérisée en ce que** les inhibiteurs sont utilisés par voie parentérale, entérale, orale, par inhalation ou sous forme de suppositoire.

9. Inhibiteur de la dipeptidyl-peptidase IV (DP IV ou CD 26) pour le traitement de l'anxiété.

10. Inhibiteur de la dipeptidyl-peptidase IV selon la revendication 9, **caractérisé en ce que** la dégradation du neuropeptide Y (NPY) localisé au SNC, endogène, est réduite.

11. Inhibiteur de la dipeptidyl-peptidase IV selon la revendication 9 ou 10, **caractérisé en ce que** l'inhibiteur est utilisé en combinaison avec le neuropeptide Y.

12. Inhibiteur de la dipeptidyl-peptidase IV selon l'une quelconque des revendications 9 à 11, **caractérisé en ce que** l'inhibiteur est présent dans un véhicule de délivrance de médicament physiologiquement compatible.

13. Inhibiteur de la dipeptidyl-peptidase IV selon l'une quelconque des revendications 9 à 12, **caractérisé en ce que** l'inhibiteur est choisi parmi des N-(N'-substitution glycyl)-2-cyanopyrrolidines, la L-*thréo*-isoleucyl-thiazolidine, la L-*allo*-isoleucyl-thiazolidine, la L-*thréo*-isoleucyl-pyrrolidine et la L-*allo*-isoleucyl-pyrrolidine.

14. Inhibiteur de la dipeptidyl-peptidase IV selon l'une quelconque des revendications 9 à 13, **caractérisé en ce que** l'inhibiteur est la L-*thréo*-isoleucyl-thiazolidine.

15. Inhibiteur de la dipeptidyl-peptidase IV selon l'une quelconque des revendications 9 à 14, **caractérisé en ce que** l'inhibiteur est formulé sous la forme d'un précurseur de formule générale A-B-C, où A représente un acide aminé, B représente une liaison chimique entre A et C ou un acide aminé, et C représente un inhibiteur stable ou instable de la dipeptidyl-peptidase IV.

16. Inhibiteur de la dipeptidyl-peptidase IV selon l'une quelconque des revendications 9 à 15, **caractérisé en ce que** l'inhibiteur est utilisé par voie parentérale, entérale, orale, par inhalation ou sous la forme d'un suppositoire.
